# EUROPEAN PATENT SPECIFICATION

(11) **EP 4 463 079 B1**
(45) Date of publication and mention of the grant of the patent: **23.09.2026**
(21) Application number: 23702892.3
(22) Date of filing: 05.01.2023
(51) Int. Cl.: A61B 17/04, A61B 5/02, A61B 90/50, A61F 2/24, A61B 90/57, A61B 34/20

(54) **BIOELECTRICAL GUIDANCE FOR ANCHORING**
BIOELEKTRISCHE FÜHRUNG ZUR VERANKERUNG
GUIDAGE BIOÉLECTRIQUE POUR L'ANCRAGE

(30) Priority: 10.01.2022 US 202263298199 P
(43) Date of publication of application: 20.11.2024
(73) Proprietor: Edwards Lifesciences Innovation (Israel) Ltd., 3079892 Caesarea (IL)
(72) Inventor: HARUSH, Lior, 4658804 Herzliya (IL); HABERMAN BROWNS, Bezalel, 3702166 Pardes Hanna (IL); SHEPS, Tal, 5401453 Givat Shmuel (IL); KAYE, Paul, 7176051 Modiin (IL); HERMAN, Yaron, 3780800 Givat Ada (IL); GALON, Aviv, 6249247 Tel Aviv (IL); HOFFER, Eran, 563007 Yahud (IL); KERET, Yuval, 6416701 Tel Aviv (IL)
(74) Representative: Eisenführ Speiser
(86) International application number: PCT/IB2023/050073
(87) International publication number: WO 2023/131886

(56) References cited:
- US-A1- 2015 119 934
- US-A1- 2015 272 734

## Description

### FIELD OF THE INVENTION

The present invention relates in general to systems and techniques for implanting an implant at or in a heart of a subject. More specifically, some applications of the present invention relate to systems and techniques for implanting an implant at or in a heart of a subject, guided by electrical signals.

### BACKGROUND

Dilation of an annulus of a heart valve may occur due to various heart conditions, such as an enlarged heart chamber or a leaking heart valve. An annuloplasty procedure may be necessary to reshape, reinforce or tighten the annulus. Annuloplasty may be performed by implanting an annuloplasty implant to re-shape and/or re-size the annulus, for example, to reduce the size of the annulus.

Tissue anchors can be used to facilitate implantation of such annuloplasty implants, such as by coupling such implants to tissue of the heart. An anchor may be driven through the implant and into the tissue while the implant is held in place. Alternatively, the anchor may be implanted before introduction of the implant, and the implant subsequently coupled to the anchor.

Tissue anchors can also be used to facilitate implantation of other cardiac implants, such as other annulus-anchored implants.

US 2015/0272734 A1 describes a catheter to be advanced toward an anatomical site, which has a proximal end and a steerable distal end. An anchor is advanced through the catheter. An anchor driver drives the anchor out of the catheter's distal end, anchoring the anchor at the site. A first constraining member engages tissue, and inhibits, after the anchor has been driven out of the catheter and before the anchoring, movement of at least the anchor driver's distal end, on a first axis between the anchor driver's distal end and a site at which the first constraining member engages the tissue. A second constraining member inhibits, after the anchor has been driven out of the catheter and before the anchoring, movement of at least the anchor driver's distal end, on a second axis.

US 2015/0119934 A1 describes a system for closing a wound created across a portion of a tissue structure, comprising a suture member having proximal and distal ends; an anchor member coupled to the distal end of the suture member; a tension retainer assembly releasably coupleable to the suture member; and a suture buttress movably intercoupled to the suture member between the anchor member and the tension retainer and configured to minimize direct sliding contact between the suture member and the tissue structure portion around the location of the suture buttress.

### SUMMARY OF THE INVENTION

The present disclosure relates, *inter alia,* to methods and systems of implanting an implant at a heart of a subject (e.g. a human subject), guided by electrophysiological signals produced by the heart - for example, using an anchor of the implant to serve as an electrode within the heart, in order to detect these electrophysiological signals.

Typically, before implanting of the implant in the heart, a controller (e.g. a surgeon and/or a data-processing system, such as a computer processor) receives information regarding the desired implantation site of the implant within the heart. Since the implant is typically attached to the heart via the anchor, it may be important for the anchor to be anchored precisely, according to the surgical plan, in order for the implant to serve the desired purpose. For example, for applications in which the implant is an annuloplasty implant, for implanting around an annulus of a valve of the heart, it is typically desirable to anchor anchors of the annuloplasty implant into the annulus - e.g. to drive the anchor through an atrial surface of the annulus. For some applications it is thus undesirable to anchor such anchors to other tissues, such as into the wall of the atrium upstream of the valve or the wall of the ventricle downstream of the valve.

In accordance with the present invention, a system for use with a heart of a subject is provided as defined in claim 1. The dependent claims define selected embodiments.

In order to intraoperatively determine whether an anchor is optimally positioned for anchoring, prior to driving the anchor into the tissue, a distal tip of the anchor may be placed against the tissue. In this position, the anchor is used as an electrode via which a data-processing system, electrically connected to the anchor, can acquire electrical signals produced by the heart. Based on this information, the data-processing system provides an indication of the location of the anchor within the heart, which the operator (e.g. physician) can use to facilitate optimal anchoring of the anchor.

The data-processing system may be adapted to associate various electrical signals with corresponding locations of the electrode within the heart (e.g. different tissues of the heart, or different locations along an atrioventricular axis of the heart). For example, a signal acquired from an anchor placed against tissue of the annulus may be different from a signal acquired from the same anchor placed against tissue of the atrium, or placed against tissue of the ventricle. If the data-processing system determines that the anchor is located satisfactorily within the heart, the anchor can then be driven into the tissue.

For some applications, the data-processing system is electronically coupled to the anchor via an anchor driver, used to drive the anchor into the tissue. For example, a wire with a connector (e.g. a crocodile clip) at its end, may extend from the data-processing system, and may be mechanically and electrically connected to (e.g. clipped onto) a part of an electrically conductive shaft of the anchor driver that is disposed outside of the subject.

For some applications, the data-processing system also receives an electrical signal (i.e. a second electrical signal) from an additional component of the implantation system (e.g. a delivery tool of the implant and/or an additional component of the implant itself) - i.e. in addition to the electrical signal from the anchor (which may be referred to as the first electrical signal). This additional component may be considered to be a second electrode, with the anchor being referred to as a first electrode. From the first and second signals, the data-processing system may derive a refined signal, which may be improved (e.g. to have a better signal-to-noise ratio) compared to the first signal alone.

For some such applications, the second electrode is not in contact with the tissue during the detection of the signals - e.g. it may be suspended in the bloodstream, within the heart. For example, the implantation system may be configured to facilitate such placement.

A reference electrode is typically placed on the subject at a distance to the heart, for example, on the skin of the subject, in order to acquire each of the first and second signals.

The above described application may be implemented during an annuloplasty procedure in which an annuloplasty implant is implanted around an annulus of a heart valve, in order to reduce valve regurgitation. The annuloplasty implant may comprise a contracting mechanism, such as a spool, in order to contract a tether of the implant once the implant has been implanted around the annulus, in order to reduce the circumference of the valve. For such an application, the contracting mechanism may serve as the second electrode. A guide member may extend, from the contracting mechanism, proximally out of the subject, e.g. in order to facilitate advancement of an adjustment tool therealong to actuate the contracting mechanism. The guide member may serve as a conductor from the contracting mechanism (serving as the second electrode). A second wire with a second connector at its end may extend from the data-processing system, and may be mechanically and electrically connected to (e.g. clipped onto) a part of the guide member that is disposed outside of the subject.

Additionally or alternatively, the above described application may be implemented by having the second electrode disposed on a distal end portion of a delivery catheter of the delivery tool, with a conductor extending proximally along the delivery catheter to an extracorporeal part of the delivery tool, where a wire may be mechanically and electrically connected to the conductor.

A technique for assessing whether an anchor has been fully driven into tissue of the heart is now described. For some applications, the anchor comprises a tissue-engaging element that is adapted to be driven into the tissue, and an anchor-head that has a tissue-facing surface that, once the tissue-engaging element has been fully driven into the tissue, contacts the tissue surface. The anchor is typically delivered to the heart and driven into the tissue using an anchor driver that is reversibly coupled to the anchor-head.

Receiving an indication that the anchor-head is in direct contact with the tissue surface may provide an indication that anchoring has been successful and/or is complete (e.g. that the anchor has been driven into the tissue to the required depth). In order to determine whether there is direct contact between the anchor-head and the tissue surface, a tissue-facing electrode disposed at the tissue-facing surface of the anchor-head serves as a detecting electrode, from which a data-processing system receives an electrical signal. Thus, once the tissue-facing surface of the anchor-head comes into direct contact with the tissue surface, the resulting electrical signal is received by the data-processing system, which responsively provides feedback that the anchor is properly implanted within the tissue. The data-processing system typically receives these electrical signals via the anchor driver.

A technique for assessing whether an anchor is being driven into tissue of the annulus is provided, in accordance with some applications. For some applications, subsequently to determining that an anchor is contacting tissue of the annulus, it is advantageous to continuously assess, during driving of the anchor into the tissue, whether the anchor is being anchored correctly (e.g. using methods similar to those described hereinabove). Different tissues of the heart may respond differently to the anchoring process, such that a data-processing system (e.g. a data-processing system as described hereinabove) may be able to provide an indication of the location and/or positioning of the anchor within the tissue, responsively to the anchoring. For example, the electrical signal detected by the anchor typically changes as the tissue-engaging element passes into solid tissue. The technique thus includes, by the anchor serving as an electrode within the heart, using the anchor to detect the electrophysiological signals produced by the heart, as the anchor is driven into the tissue, and responsively to the electrical signal, determining whether the anchor is being driven into the tissue as desired.

For some applications, one or more of the techniques described herein is augmented by electrical-sensing-based determination of an orientation of the distal part of the delivery system - e.g. via sensing of endogenous or exogenous electrical signals.

There is therefore provided, in accordance with some applications of the present invention, a system for use with a heart of a subject, the system including and anchor, a delivery tool, and a data-processing system. The delivery tool may be configured to deliver the anchor to the heart of the subject, and/or to drive the anchor into tissue of the heart. The data-processing system may be adapted to, subsequently to the delivery tool delivering the anchor to the heart, and prior to the delivery tool driving the anchor into the tissue:
receive, via the delivery tool, a first electrical signal from the anchor, at the tissue, the anchor serving as an electrode;
receive, via the delivery tool, a second electrical signal from a distal part of the delivery tool, within the heart,
responsively to both the first signal and the second signal, determine a location of the anchor within the heart, and/or
provide an output indicative of the location.

For some applications, the data-processing system is adapted to receive the first electrical signal, receive the second electrical signal, determine the location, and/or provide the output, prior to the delivery tool driving the anchor into the tissue.

For some applications, the anchor is a helical anchor.

For some applications,:
the data processing system is adapted to, prior to receiving the first signal:
   receive, via the delivery tool, a first initial electrical signal from the anchor, within the subject but distanced from the tissue, the anchor serving as the electrode,
   receive, via the delivery tool, a second initial electrical signal from the distal part of the delivery tool, within the subject but distanced from the tissue, and/or
   responsively to the first initial electrical signal and the second initial electrical signal, assigning the subject to a category, and/or
the data-processing signal is adapted to determine the location responsively to the first electrical signal, the second electrical signal, and the category.

For some applications, the data-processing system is adapted to receive the first initial electrical signal and the second initial electrical signal prior to the delivery tool delivering the anchor to the heart.

For some applications,
the output is indicative of the location and an orientation of the anchor within the heart, and/or
the data-processing system is further adapted to, responsively to both the first signal and the second signal:
   determine the orientation of the anchor within the heart, and/or
   provide the output indicative of the location and the orientation of the anchor within the heart.

For some applications:
the orientation is an orientation of the anchor with respect to an atrioventricular axis of the heart,
the output is indicative of the location and of the orientation with respect to the atrioventricular axis, and/or
the data-processing system is adapted to, responsively to both the first signal and the second signal:
   determine the orientation of the anchor with respect to the atrioventricular axis, and/or
   provide the output indicative of the location and of the orientation of the anchor with respect to the atrioventricular axis.

For some applications:
the orientation is an orientation of the anchor with respect to a tissue surface of the heart,
the output is indicative of the location and of the orientation with respect to the tissue surface, and/or
the data-processing system is adapted to, responsively to both the first signal and the second signal:
   determine the orientation of the anchor with respect to the tissue surface, and/or
   provide the output indicative of the location and of the orientation of the anchor with respect to tissue surface.

For some applications:
the heart has an atrium, a ventricle, and a valve therebetween, the valve having an annulus, the heart defining an atrioventricular axis extending from the atrium to the ventricle,
the location of the anchor is a location of the anchor along the atrioventricular axis,
the data-processing system is adapted to, subsequently to the delivery tool delivering the anchor to the heart, and prior to the delivery tool driving the anchor into the tissue, responsively to both the first signal and the second signal, determine the location of the anchor along the atrioventricular axis,
the output is indicative of the location of the anchor along the atrioventricular axis, and
the data-processing system is adapted to provide the output indicative of the location of the anchor along the atrioventricular axis.

For some applications, the data-processing system is adapted to, subsequently to the delivery tool delivering the anchor to the heart, and prior to the delivery tool driving the anchor into the tissue:
receive the first signal and the second signal concurrently, and
determine the location of the anchor within the heart responsively to the concurrently received first signal and second signal.

For some applications, the data-processing system is adapted to determine the location of the anchor within the heart by determining a difference between the first signal and the second signal.

For some applications, the data-processing system is adapted to, subsequently to providing the output indicative of the location of the anchor within the heart, and while the anchor is driven into the tissue:
responsively to at least one signal selected from the group consisting of the first signal, and the second signal, determine a response of the tissue to the anchoring, and
responsively to the determined response, provide an output indicative of at least one of (i) a depth of the anchor within the tissue, and (ii) the location of the anchor within the heart of the subject.

For some applications, the anchor has a tissue-engaging element and a head, and the data-processing system is adapted to, subsequently to providing the output indicative of the location of the anchor within the heart, and while the anchor is driven into the tissue:
responsively to both the first signal and the second signal, determine a response of the tissue to the anchoring,
receive, from the head of the anchor, a head-contact signal indicative of contact between the head of the anchor and the tissue, and
responsively to (i) the determined response, and (ii) the head-contact signal, determine an angle of attack of the anchor with respect to the tissue.

For some applications:
the delivery tool includes a driver, reversibly engageable with the anchor, and adapted to drive the anchor into the tissue while engaged with the anchor, and
the data-processing system is adapted to receive the first signal via the driver.

For some applications, the driver is disengageable from the anchor within the heart.

For some applications:
the driver includes:
   a tip, reversibly engageable with the anchor, and
   a shaft extending from a proximal end of the driver to the tip, configured to transfer torque from the proximal end of the driver to the tip, and being electrically conductive, and
the data-processing system is adapted to receive the first signal via the shaft.

For some applications:
the driver includes:
   a tip, reversibly engageable with the anchor,
   a shaft extending from a proximal end of the driver to the tip, configured to transfer torque from the shaft, and
   a rod, extending through the shaft to the tip, configured to control engagement of the tip with the anchor, and being electrically conductive, and
the data-processing system is adapted to receive the first signal via the rod.

For some applications:
the system further includes an adjustment tool, and an annuloplasty implant that includes:
   the anchor, configured to anchor the implant to the tissue,
   a tether, and
   a contracting mechanism configured to, upon actuation of the contracting mechanism, contract the implant, and
the distal part of the delivery tool includes a distal part of a guide member of the delivery tool, mechanically connected to the contracting mechanism, the guide member extending proximally from the contracting mechanism, and configured to guide the adjustment tool transluminally to the contracting mechanism subsequently to anchoring of the implant to the tissue, the adjustment tool being configured to actuate the contracting mechanism, and
the data-processing system is adapted to, subsequently to the delivery tool delivering the anchor to the heart, and prior to the delivery tool driving the anchor into the tissue, receive the second electrical signal from the distal part of the guide member that is mechanically connected to the contracting mechanism.

For some applications, the distal part of the guide member is mechanically and electrically connected to the contracting mechanism, and the data-processing system is adapted to, subsequently to the delivery tool delivering the anchor to the heart, and prior to the delivery tool driving the anchor into the tissue, receive the second electrical signal from the distal part of the guide member that is mechanically and electrically connected to the contracting mechanism.

For some applications, the system further includes a sensing device that includes the data-processing system and a connector, the connector being electrically and mechanically connectable to a proximal part of the guide member in a manner that configures the data-processing system to receive the second signal from the distal part of the guide member via the connector.

For some applications, the connector is a crocodile clip that is clippable onto the proximal part of the guide member.

For some applications, the distal part of the guide member is mechanically and electrically connected to the contracting mechanism, and the connector is electrically and mechanically connectable to the proximal part of the guide member in a manner that configures the data-processing system to receive the second signal from the contracting mechanism via the guide member and the connector.

For some applications, subsequently to actuation of the contracting mechanism by the adjustment tool, the guide member is intracorporeally disconnectable from the contracting mechanism such that the contracting mechanism, within the heart, becomes electrically isolated from the data-processing system.

For some applications, the anchor defines a distal tip, adapted to penetrate tissue of the heart, and the data-processing system is adapted to receive the first signal while the distal tip of the anchor is placed against the tissue.

For some applications, the data-processing system is adapted to receive the first signal while the distal tip of the anchor is placed against a surface of the tissue of the heart, not penetrating the tissue.

For some applications, the system further includes a sleeve adapted to be anchored to the tissue by the anchor, and the data-processing system is adapted to receive the first signal while the sleeve is sandwiched between the distal tip and a surface of the tissue.

For some applications, the data-processing system is adapted to receive the first signal while the distal tip of the anchor is disposed within the tissue, having penetrated the tissue.

For some applications, the system further includes a sensing device that includes the data-processing system and a first connector, the first connector being electrically and mechanically connectable to a proximal part of the delivery tool, thereby configuring the data-processing system to receive the first signal from the anchor via the first connector.

For some applications, the first connector is a crocodile clip that is clippable onto the proximal part of the delivery tool.

For some applications, the sensing device further includes a second connector, the second connector being electrically and mechanically connectable to a proximal portion of the delivery tool, thereby configuring the data-processing system to receive the second signal from the distal part of the delivery tool via the second connector.

For some applications, the second connector is a crocodile clip that is clippable onto the proximal part of the delivery tool.

For some applications, the second connector is an electronic snap.

For some applications:
the heart has an atrium, a ventricle, and a valve therebetween, the valve having an annulus,
the data-processing system is adapted to, subsequently to the delivery tool delivering the anchor to the heart, and prior to the delivery tool driving the anchor into the tissue, responsively to both the first signal and the second signal, determine whether the location of the anchor is at the annulus,
the output is indicative of whether the location of the anchor is at the annulus, and
the data-processing system is adapted to provide the output indicative of whether the location of the anchor is at the annulus.

For some applications:
the data-processing system is further adapted to, subsequently to the delivery tool delivering the anchor to the heart, and prior to the delivery tool driving the anchor into the tissue, responsively to both the first signal and the second signal, determine whether the location of the anchor is at the atrium or at the ventricle,
the output is indicative of whether the location of the anchor is at the annulus, at the atrium, or at the ventricle, and
the data-processing system is adapted to provide the output indicative of whether the location of the anchor is at the annulus, at the atrium, or at the ventricle.

For some applications:
the electrode is a first electrode, the anchor serving as the first electrode, and
the distal part of the delivery tool includes a second electrode, the data-processing system being adapted to receive the second electrical signal from the second electrode.

For some applications, the second electrode is disposed on a lateral wall of a catheter of the delivery tool.

For some applications:
the catheter includes a wire that extends, from the second electrode, along the catheter to an extracorporeal portion of the catheter; and
the data-processing system is adapted to receive the second electrical signal from the second electrode via the wire.

For some applications:
at the extracorporeal portion of the catheter, the wire terminates at a connector; and
the data-processing system is electrically and mechanically connectable to the second electrode via the connector.

For some applications, the data-processing system is adapted to receive the second signal while the second electrode is suspended in a bloodstream of the heart.

For some applications, the data-processing system is adapted to receive the second signal while the second electrode does not contact the tissue of the heart.

For some applications, the system further includes a reference electrode, adapted to be placed outside of the heart of the subject, and the data-processing system is configured to determine the location, facilitated by the reference electrode.

For some applications, the reference electrode is a skin electrode, adapted to be placed on skin of the subject.

There is further provided, in accordance with some applications, a system for use with a heart of a subject, the system comprising an anchor, a delivery tool, and a data-processing system.

The delivery tool may be configured to deliver the anchor to the heart of the subject, and to drive the anchor into tissue of the heart.

The data-processing system may be adapted to be electrically connected to the delivery tool such that the data-processing system receives (i) via the delivery tool, a first electrical signal from the anchor, and/or (ii) via the delivery tool, a second electrical signal from a distal part of the delivery tool.

While the anchor is coupled to the delivery tool within the heart, the data-processing system may be configured to, responsively to both the first signal and the second signal, determine a location of the anchor within the heart, and provide an output indicative of the location.

There is further provided, in accordance with some applications of the present invention, a system for use with a heart of a subject, the system including an implant, a driver, and a data-processing system.

The implant may include an anchor, a tether, couplable to the anchor, and a contracting mechanism, for applying tension to the tether.

The driver may be configured to deliver the anchor to the heart of the subject, and to drive the anchor into tissue of the heart.

For some applications, the data-processing system is adapted to receive the first electrical signal, receive the second electrical signal, determine the location, and/or provide the output, prior to the delivery tool driving the anchor into the tissue.

The data-processing system may be adapted to, subsequently to the driver delivering the anchor to the heart, and prior to the driver driving the anchor into the tissue:
receive, via the driver, a first electrical signal from the anchor serving, at the tissue, as a first electrode, the driver providing an anchor-to-data-processing system electrical connection,
receive, via a second electrical connection, a second electrical signal from the contracting mechanism, serving, within the heart, as a second electrode,
responsively to both the first signal and the second signal, determine a location of the anchor within the heart, and/or
provide an output indicative of the location.

For some applications, the data-processing system is adapted to, subsequently to providing the output indicative of the location of the anchor within the heart, and while the anchor is driven into the tissue:
responsively to at least one signal selected from the group consisting of the first signal, and the second signal, determine a response of the tissue to the anchoring, and
responsively to the determined response, provide an output indicative of at least one of (i) a depth of the anchor within the tissue, and (ii) the location of the anchor within the heart of the subject.

For some applications, the anchor has a tissue-engaging element and a head, and the data-processing system is adapted to, subsequently to providing the output indicative of the location of the anchor within the heart, and while the tissue-engaging element is driven into the tissue:
responsively to both the first signal and the second signal, determine a response of the tissue to the anchoring,
receive, from the head of the anchor, a head-contact signal indicative of contact between the head of the anchor and the tissue, and
responsively to (i) the determined response, and (ii) the head-contact signal, determine an angle of attack of the anchor with respect to the tissue.

For some applications:
the heart has an atrium, a ventricle, and a valve therebetween, the valve having an annulus, the heart defining an atrioventricular axis extending from the atrium to the ventricle,
the location of the anchor is a location of the anchor along the atrioventricular axis,
the data-processing system is adapted to, subsequently to the driver delivering the anchor to the heart, and prior to the driver driving the anchor into the tissue, responsively to both the first signal and the second signal, determine the location of the anchor along the atrioventricular axis,
the output is indicative of the location of the anchor along the atrioventricular axis, and
the data-processing system is adapted to provide the output indicative of the location of the anchor along the atrioventricular axis.

For some applications, the data-processing system is adapted to receive the second signal while the contracting mechanism is suspended in a bloodstream of the heart.

For some applications, the data-processing system is adapted to receive the second signal while the contracting mechanism does not contact the tissue of the heart.

For some applications, the data-processing system is adapted to, subsequently to the driver delivering the anchor to the heart, and prior to the driver driving the anchor into the tissue:
receive the first signal and the second signal concurrently, and
determine the location of the anchor within the heart responsively to the concurrently received first signal and second signal.

For some applications, the data-processing system is adapted to determine the location of the anchor within the heart by determining a difference between the first signal and the second signal.

For some applications, the system further includes a sensing device that includes the data-processing system and a first connector, the first connector being electrically and mechanically connectable to a proximal part of the driver, thereby configuring the data-processing system to receive the first signal from the anchor via the first connector.

For some applications, the first connector is a crocodile clip that is clippable onto the proximal part of the driver.

For some applications, the first connector is an electronic snap.

For some applications, the system further includes:
an adjustment tool, and
a guide member, mechanically connected to the contracting mechanism, the guide member extending proximally from the contracting mechanism, and configured to guide the adjustment tool transluminally to the contracting mechanism subsequently to anchoring of the implant to the tissue, the adjustment tool being configured to actuate the contracting mechanism,
and the data-processing system is adapted to receive the second electrical signal from the contracting mechanism via the guide member.

For some applications, the system further includes a sensing device that includes the data-processing system and a connector, the connector being electrically and mechanically connectable to a proximal part of the guide member in a manner that configures the data-processing system to receive the second signal from the contracting mechanism via the guide member and the connector.

For some applications, the connector is a crocodile clip that is clippable onto the proximal part of the guide member.

For some applications, subsequently to actuation of the contracting mechanism by the adjustment tool, the guide member is intracorporeally disconnectable from the contracting mechanism such that the contracting mechanism, within the heart, becomes electrically isolated from the data-processing system.

For some applications, the driver is disengageable from the anchor within the heart.

For some applications:
the driver includes:
   a tip, reversibly engageable with the anchor, and
   a shaft extending from a proximal end of the driver to the tip, configured to transfer torque from the proximal end of the driver to the tip, and being electrically conductive, and
the data-processing system is adapted to receive the first signal via the shaft.

For some applications:
the driver includes:
   a tip, reversibly engageable with the anchor,
   a shaft extending from a proximal end of the driver to the tip, configured to transfer torque from the shaft, and
   a rod, extending through the shaft to the tip, configured to control engagement of the tip with the anchor, and being electrically conductive, and
the data-processing system is adapted to receive the first signal via the rod.

For some applications, the anchor defines a distal tip, adapted to penetrate tissue of the heart, and the data-processing system is adapted to receive the first signal while the distal tip of the anchor is placed against the tissue.

For some applications, the data-processing system is adapted to receive the first signal while the distal tip of the anchor is placed against a surface of the tissue of the heart, not penetrating the tissue.

For some applications, the implant further includes a sleeve adapted to be anchored to the tissue by the anchor, and the data-processing system is adapted to receive the first signal while the sleeve is sandwiched between the distal tip and a surface of the tissue.

For some applications, the data-processing system is adapted to receive the first signal while the distal tip of the anchor is disposed within the tissue, having penetrated the tissue.

For some applications:
the heart has an atrium, a ventricle, and a valve therebetween, the valve having an annulus,
the data-processing system is adapted to, subsequently to the driver delivering the anchor to the heart, and prior to the driver driving the anchor into the tissue, responsively to both the first signal and the second signal, determine whether the location of the anchor is at the annulus,
the output is indicative of whether the location of the anchor is at the annulus, and
the data-processing system is adapted to provide the output indicative of whether the location of the anchor is at the annulus.

For some applications:
the data-processing system is further adapted to, subsequently to the driver delivering the anchor to the heart, and prior to the driver driving the anchor into the tissue, responsively to both the first signal and the second signal, determine whether the location of the anchor is at the atrium or at the ventricle,
the output is indicative of whether the location of the anchor is at the annulus, at the atrium, or at the ventricle, and
the data-processing system is adapted to provide the output indicative of whether the location of the anchor is at the atrium, at the annulus, or at the ventricle.

For some applications, the system further includes a reference electrode, adapted to be placed outside of the heart of the subject, and the data-processing system is configured to determine the location, facilitated by the reference electrode.

For some applications, the reference electrode is a skin electrode, adapted to be placed on skin of the subject.

There is further provided, in accordance with some applications of the present invention, a system for use at a heart of a subject. The system may include an anchor which may include a tissue-engaging element and an anchor-head having a tissue-facing electrode.

A driver may be adapted to, via engagement with the anchor-head, place the tissue-facing electrode in contact with a surface of a tissue of the heart by driving the tissue-engaging element into the tissue.

A data-processing system may be adapted to receive, via the driver, an electrical signal from the tissue-facing electrode, the electrical signal being indicative of contact between the tissue-facing electrode and the tissue, and/or responsively to the electrical signal, providing an indication of contact between the anchor-head and the tissue surface.

For some applications, the driver is adapted to drive the tissue-engaging element into the tissue such that the tissue-facing electrode becomes pressed against the surface of the tissue without penetrating the tissue.

For some applications, the anchor is electrically disconnectable from the data-processing system subsequently to driving the tissue-engaging element into the tissue, by disengaging the driver from the anchor-head.

For some applications, the anchor-head defines a tissue-facing surface, and the tissue-facing surface serves as the tissue-facing electrode.

For some applications, the driver is adapted to drive the tissue-engaging element into the tissue such that the tissue-facing surface becomes pressed against the surface of the tissue without penetrating the tissue.

For some applications, the anchor-head is formed from an electrically conductive material, and the data-processing system is adapted to receive the electrical signal from the tissue-facing surface, via the anchor-head.

For some applications, the tissue-engaging element is electrically isolated from the data-processing system.

For some applications, the tissue-engaging element is electrically isolated from the data-processing system by the tissue-engaging element being electrically isolated from the tissue-facing electrode.

For some applications, the anchor-head includes:
an inner core, the tissue-engaging element being coupled to the inner core;
an outer layer with which the driver is engageable, the outer layer serving as the tissue-facing electrode; and
an insulating layer electrically insulating the inner core from the outer layer.

For some applications, the system further includes a sensing device that includes the data-processing system and a connector, the connector being electrically and mechanically connectable to a proximal part of the driver, thereby configuring the data-processing system to receive the electrical signal from the tissue-facing electrode via the connector.

For some applications, the connector is a crocodile clip that is clippable onto the proximal part of the driver.

For some applications, the connector is an electronic snap.

For some applications, the driver is disengageable from the anchor-head within the heart.

For some applications:
the driver includes:
   a tip, reversibly engageable with the anchor-head, and
   a shaft extending from a proximal end of the driver to the tip, configured to transfer torque from the proximal end of the driver to the tip, and being electrically conductive, and
the data-processing system is adapted to receive the electrical signal via the shaft.

For some applications:
the driver includes:
   a tip, reversibly engageable with the anchor-head,
   a shaft extending from a proximal end of the driver to the tip, configured to transfer torque from the shaft, and
   a rod, extending through the shaft to the tip, configured to control engagement of the tip with the anchor-head, and being electrically conductive, and
the data-processing system is adapted to receive the electrical signal via the rod.

For some applications, the system further includes a reference electrode, adapted to be placed outside of the heart of the subject, and the data-processing system is configured to determine contact between the anchor-head and the tissue surface, facilitated by the reference electrode.

For some applications, the reference electrode is a skin electrode, adapted to be placed on skin of the subject.

There is further provided, in accordance with some applications of the present invention, a system for use at a heart of a subject, the system including an anchor, a driver, and a data-processing system.

The anchor may include a tissue-engaging element, and an anchor-head.

The driver may be adapted to drive the tissue-engaging element into tissue of the heart, while (i) electrically connected with an electrode at a tissue-facing surface of the anchor-head, and (ii) electrically isolated from the tissue-engaging element.

The data-processing system may be adapted to, during driving of the tissue-engaging element into the tissue (i) receive, via the driver, an electrical signal from the electrode, and/or (ii) provide an indication of contact between the anchor-head and a surface of the tissue, responsively to the electrical signal.

For some applications, the data-processing system is adapted to, responsively to the electrical signal, determine an angle of attack of the anchor with respect to the tissue.

There is further provided, in accordance with some applications of the present invention, a device for use with (i) an anchor, and (ii) a delivery tool including an anchor driver adapted to transluminally drive the anchor into tissue of a heart of a subject.

The device may include a first wire, electrically and mechanically connectable to a proximal part of the anchor driver in a manner that electrically connects the device to the anchor via the first wire and engagement of a distal part of the anchor driver with the anchor, and a second wire electrically and mechanically connectable to a proximal part of the delivery tool in a manner that electrically connects the device to a distal part of the delivery tool.

A data-processing system may be adapted to, while (a) the distal part of the anchor driver is engaged with the anchor, and (b) the anchor and the distal part of the delivery tool are disposed within the heart (i) responsively to electrical sensing via the first wire and the second wire, determine a location of the anchor within the heart, and/or (ii) provide an output indicative of the location.

For some applications, the device further includes a third wire electrically and mechanically connected to a reference electrode adapted to be placed outside of the heart of the subject, and the data-processing system is adapted to:
receive a first electrical signal between (i) the reference electrode and (ii) the anchor serving as a first electrode within the heart;
receive a second electrical signal between (i) the reference electrode, and (ii) the distal part of the delivery tool;
responsively to the first signal and the second signal, determine the location of the anchor within the heart; and
provide the output indicative of the location.

For some applications, the data-processing system is adapted to:
via the electrical sensing, receive an electrical signal between (i) the anchor, and (ii) the distal part of the delivery tool; and
determine the location of the anchor within the heart responsively to the electrical signal; and
provide the output indicative of the location.

For some applications, the device further includes a third wire electrically and mechanically connected to a reference electrode adapted to be placed outside of the heart of the subject, and the data-processing system is adapted to:
receive a first electrical signal between (i) the reference electrode and (ii) the anchor serving as a first electrode within the heart;
receive a second electrical signal between (i) the anchor, and (ii) the distal part of the delivery tool;
determine the location of the anchor within the heart responsively to the first signal and the second signal; and
provide the output indicative of the location.

There is further provided, in accordance with some applications of the present invention, a system for use at a heart of a subject, the system including an implant, an anchor, a driver, and a data-processing system.

The implant may be adapted to reduce regurgitation of a valve of the heart.

The anchor may be for securing the implant to tissue of the heart. The anchor may include a tissue-engaging element, and an anchor-head.

The driver may be adapted to anchor the anchor to the tissue by driving the tissue-engaging element into the tissue.

The data-processing system may be adapted to, while the tissue-engaging element is being driven into the tissue:
receive, via the driver, an electrical signal from the anchor, the electrical signal being indicative of a response of the tissue to the anchoring, and/or
responsively to the electrical signal, provide an indication of the location of the anchor within the heart of the subject.

For some applications, the anchor is a helical anchor.

For some applications:
the electrical signal is a first electrical signal,
the system further includes:
   a delivery tool that includes the driver, and that is adapted to deliver the anchor to the heart of the subject, and
   a second electrode disposed at a distal part of the delivery tool, and
the data-processing system is adapted to, while the tissue-engaging element is being driven into the tissue:
   receive a second electrical signal from the second electrode, and
   responsively to the first signal and the second signal, provide the indication of the location of the anchor within the heart of the subject.

For some applications:
the heart has an atrium, a ventricle, and a valve therebetween, the valve having an annulus, the heart defining an atrioventricular axis extending from the atrium to the ventricle,
the location of the anchor is a location of the anchor along the atrioventricular axis,
the data-processing system is adapted to, during the tissue-engaging element being driven into the tissue, responsively to the electrical signal, determine the location of the anchor along the atrioventricular axis,
the output is indicative of the location of the anchor along the atrioventricular axis, and
the data-processing system is adapted to provide the output indicative of the location of the anchor along the atrioventricular axis.

For some applications, the data-processing system is further adapted to, responsively to the electrical signal, determine an angle of attack of the anchor with respect to the tissue.

For some applications, the data-processing system is further adapted to, responsively to the electrical signal, determine a depth of the anchor within the tissue.

For some applications, the system further includes a sensing device that includes the data-processing system and a connector, the connector being electrically and mechanically connectable to a proximal part of the driver, thereby configuring the data-processing system to receive the electrical signal from the anchor via the connector.

For some applications, the connector is a crocodile clip that is clippable onto the proximal part of the driver.

For some applications, the first connector is an electronic snap.

For some applications:
the electrical signal is a first electrical signal,
the system further includes:
   an adjustment tool,
   a delivery tool including the driver, and adapted to deliver the anchor to the heart of the subject, and
the implant further includes:
   a tether, and
   a contracting mechanism configured to, upon actuation of the contracting mechanism, contract the implant,
a distal part of the contracting mechanism is electrically and mechanically connected to a guide member that extends proximally from the contracting mechanism, the guide member being configured to guide the adjustment tool transluminally to the contracting mechanism subsequently to anchoring of the implant to the tissue, the adjustment tool being configured to actuate the contracting mechanism, and
the data-processing system is adapted to, during the tissue-engaging element being driven into the tissue:
   receive a second electrical signal from the contracting mechanism via the guide member, and
   responsively to the first signal and the second signal, provide the indication of the location of the anchor within the heart of the subject.

For some applications, the system further includes a sensing device that includes the data-processing system and a connector, the connector being electrically and mechanically connectable to a proximal part of the guide member in a manner that configures the data-processing system to receive the second signal from the distal part of the guide member via the connector.

For some applications, subsequently to actuation of the contracting mechanism by the adjustment tool, the guide member is intracorporeally disconnectable from the contracting mechanism such that the contracting mechanism, within the heart, becomes electrically isolated from the data-processing system.

For some applications, the data-processing system is adapted to receive the second signal while the contracting mechanism does not contact the tissue of the heart.

For some applications, the data-processing system is adapted to, during the tissue-engaging element being driven into the tissue:
receive the first signal and the second signal concurrently, and
determine the location of the anchor within the heart responsively to the concurrently received first signal and second signal.

For some applications, the driver is disengageable from the anchor within the heart.

For some applications:
the driver includes:
   a tip, reversibly engageable with the anchor, and
   a shaft extending from a proximal end of the driver to the tip, configured to transfer torque from the proximal end of the driver to the tip, and being electrically conductive, and
the data-processing system is adapted to receive the electrical signal via the shaft.

For some applications:
the driver includes:
   a tip, reversibly engageable with the anchor,
   a shaft extending from a proximal end of the driver to the tip, configured to transfer torque from the shaft, and
   a rod, extending through the shaft to the tip, configured to control engagement of the tip with the anchor, and being electrically conductive, and
the data-processing system is adapted to receive the electrical signal via the rod.

For some applications:
the heart has an atrium, a ventricle, and a valve therebetween, the valve having an annulus and a leaflet,
the data-processing system is adapted to, during the tissue-engaging element being driven into the tissue, responsively to the electrical signal, determine whether the location of the anchor is at the annulus,
the output is indicative of whether the location of the anchor is at the annulus, and
the data-processing system is adapted to provide the output indicative of whether the location of the anchor is at the annulus.

For some applications:
the data-processing system is further adapted to, during the tissue-engaging element being driven into the tissue, responsively to the electrical signal, determine whether the location of the anchor is at the atrium or at the ventricle,
the output is indicative of whether the location of the anchor is at the annulus, at the atrium, or at the ventricle, and
the data-processing system is adapted to provide the output indicative of whether the location of the anchor is at the atrium, at the annulus, at the leaflet, or at the ventricle.

For some applications, the system further includes a reference electrode, adapted to be placed outside of the heart of the subject, and the data-processing system is configured to determine the location, facilitated by the reference electrode.

For some applications, the reference electrode is a skin electrode, adapted to be placed on skin of the subject.

There is further provided, in accordance with some applications of the present invention, a system for use at a heart of a subject, the system including an implant, an anchor, a driver, and a data-processing system.

The implant may be adapted to reduce regurgitation of a valve of the heart.

The anchor may be for securing the implant to tissue of the heart. The anchor may include a tissue-engaging element, and an anchor-head.

The driver may be adapted to anchor the anchor to the tissue by driving the tissue-engaging element into the tissue.

The data-processing system may be adapted to, during the tissue-engaging element being driven into the tissue:
receive, via the driver, an electrical signal from the anchor, the electrical signal
being indicative of a response of the tissue to the anchoring, and/or
responsively to the electrical signal, provide an indication of a depth of the tissue-engaging element within the tissue.

For some applications:
the electrical signal is a first electrical signal,
the data-processing system is adapted to:
   receive, via the driver, a head-contact signal from the anchor-head, the head-contact signal being an electrical signal indicative of contact between the anchor-head and a surface of the tissue, and
   responsively to (i) the first electrical signal, and (ii) the head-contact signal, determine an angle of attack of the anchor with respect to the tissue.

For some applications, the data-processing system is adapted to, responsively to the angle of attack, provide an indication of the angle of attack.

For some applications, the data-processing system is adapted to:
determine whether the angle of attack falls outside of a predetermined angle-of-attack range, and
responsively, provide an output indicative of the angle of attack falling outside of the predetermined angle-of-attack range.

There is further provided, in accordance with some applications a system for use with an anchor and for use at a heart of a subject, the system including:
a delivery tool:
   having a proximal part, and a distal part that is transluminally advanceable to the heart,
   configured to transluminally deliver the anchor to the heart, and to drive the anchor into tissue of the heart, and
   including, at the distal part, a first electrode and a second electrode; and/or
a data-processing system electrically connectable to the proximal part of the delivery tool and including means for carrying out a method including:
   receiving a first electrical signal from the first electrode,
   receiving a second electrical signal from the second electrode,
   responsively both to the first signal and to the second signal, determining an orientation of the distal part within the heart, and
   providing an output indicative of the orientation.

For some applications, the first electrical signal is an endogenous electrical signal, and the data-processing system is configured to receive the endogenous electrical signal from the first electrode.

For some applications, the first electrical signal is an exogenous electrical signal, the delivery tool is configured to apply the exogenous electrical signal, and the data-processing system is configured to receive the exogenous electrical signal from the first electrode.

For some applications, the first electrode and the second electrode are distributed axially along the distal part.

For some applications, the first electrode and the second electrode are distributed circumferentially around the distal part.

For some applications:
the delivery tool includes, at the distal part, a third electrode, and/or
the method further includes receiving a third electrical signal from the third electrode, and/or
determining the orientation includes determining the orientation responsively to the first signal, to the second signal, and to the third signal.

For some applications:
the method includes:
   responsively to the first signal, determining a position of the first electrode within the heart, and/or
   responsively to the second signal, determining a position of the second electrode within the heart, and/or
determining the orientation includes determining the orientation responsively to the position of the first electrode and to the position of the second electrode.

For some applications, determining the orientation includes determining the orientation responsively to (a) a difference between the position of the first electrode within the heart and the position of the second electrode within the heart, and (b) a distance between (i) a first electrode-site at which the first electrode is disposed on the distal part, and (ii) a second electrode-site at which the second electrode is disposed on the distal part.

For some applications:
the position of the first electrode is a position of the first electrode along an atrioventricular axis of the heart, and/or
determining the orientation includes determining the orientation responsively to the position of the first electrode along the atrioventricular, and to the position of the second electrode.

For some applications:
the position of the second electrode is a position of the second electrode along the atrioventricular axis of the heart, and/or
determining the orientation includes determining the orientation responsively to the position of the first electrode along the atrioventricular axis, and to the position of the second electrode along an atrioventricular axis.

For some applications:
the position of the first electrode is a proximity of the first electrode to an intracardial tissue surface, and/or
determining the orientation includes determining the orientation responsively to the proximity of the first electrode to the intracardial tissue surface, and to the position of the second electrode.

For some applications:
the position of the second electrode is a proximity of the second electrode to the intracardial tissue surface, and/or
determining the orientation includes determining the orientation responsively to the proximity of the first electrode to the intracardial tissue surface, and to the proximity of the second electrode to the intracardial tissue surface.

For some applications:
the orientation is an orientation of the distal part with respect to an atrioventricular axis of the heart,
the output is indicative of the orientation with respect to the atrioventricular axis,
determining the orientation includes determining the orientation with respect to the atrioventricular axis, and/or
providing the output includes providing the output indicative of the orientation with respect to the atrioventricular axis.

For some applications:
the orientation is an orientation of the distal part with respect to a tissue surface of the heart,
the output is indicative of the orientation with respect to the tissue surface, and/or
the data-processing system is adapted to, responsively to both the first signal and the second signal:
   determine the orientation with respect to the tissue surface, and/or
   provide the output indicative of the orientation with respect to tissue surface.

For some applications:
the output is indicative of the orientation and a location of the distal part within the heart,
the data-processing system is further adapted to, responsively to both the first signal and the second signal:
   determine the location of the distal part within the heart, and/or
   provide the output indicative of the orientation and the location of the distal part within the heart.

For some applications:
receiving a first electrical signal from a first electrode that is disposed at a distal part of a delivery tool within a heart of a subject;
receiving a second electrical signal from a second electrode, that is disposed at the distal part of the delivery tool within the heart;
responsively both to the first signal and to the second signal, determining an orientation of the distal part within the heart, and/or
providing an output indicative of the orientation.

For some applications, the first electrical signal is an endogenous electrical signal, and receiving the first electrical signal includes receiving the endogenous electrical signal.

For some applications, the first electrical signal is an exogenous electrical signal, the method further includes applying the exogenous electrical signal, and receiving the first electrical signal includes receiving the exogenous electrical signal.

For some applications:
the method further includes, prior to receiving the first signal:
   receiving a first initial electrical signal from the first electrode, disposed within the subject but outside of the heart;
   receiving a second initial electrical signal from the second, disposed within the subject but outside of the heart; and/or
   responsively to the first initial electrical signal and the second initial electrical signal, assigning the subject to a category, and/or
determining the orientation includes determining the orientation responsively to the first electrical signal, the second electrical signal, and the category.

There is further provided, in accordance with some applications, a data-processing apparatus including means for carrying out the steps of the method.

There is further provided, in accordance with some applications, a computer program including instructions which, when the program is executed by a computer, cause the computer to carry out the method.

There is further provided, a computer-readable medium having stored thereon the computer program.

There is further provided, in accordance with some applications of the present invention, a computer-implemented method, including:
receiving a first electrical signal from an anchor in contact with tissue within a heart of a subject;
receiving a second electrical signal from a second electrode suspended within a bloodstream of the heart

For some applications, the method further includes receiving a third electrical signal from a reference electrode outside of the heart of the subject.

For some applications, the method further includes calculating, responsively to the first signal and the second signal, a refined signal, the refined signal being representative of a difference between the first signal and the second signal.

For some applications, the refined signal is responsive to the first signal, the second signal, and the third signal.

The method may further include using the refined signal to provide an indication of a location of the anchor within the heart.

For some applications:
the method further includes, prior to receiving any of the first electrical signal, the second electrical signal, and the third electrical signal:
   receiving an initial signal from one or more of the anchor and the second electrode; and/or
   responsively to the initial signal, assigning the subject to a category, and/or
calculating the refined signal includes calculating the refined signal responsively to the first signal, the second signal, the third signal, and the category.

For some applications:
the method further includes, prior to receiving any of the first electrical signal, the second electrical signal, and the third electrical signal:
   receiving an initial signal from one or more of the anchor and the second electrode; and/or
   responsively to the initial signal, assigning the subject to a category, and/or
using the refined signal to provide the indication includes determining the indication responsively to the refined signal and to the category.

There is further provided, in accordance with some applications of the present invention, a computer-implemented method for use with a heart of a subject, the method including:
receiving a first electrical signal from a tissue-engaging element of an anchor in contact with tissue of a heart of a subject;
receiving a second electrical signal from an anchor-head of the anchor in contact with tissue of the heart of the subject;
responsively to the first signal, providing an indication of a location of the anchor within the heart; and
responsively to the second signal, providing an indication of contact between the anchor-head and the tissue.

There is further provided, in accordance with some applications of the present invention, a computer-implemented method for use with a heart of a subject, the method including:
receiving an electrical signal from a tissue-engaging element of an anchor in contact with tissue of the heart;
prior to the tissue-engaging element being driven into the tissue, and while the tissue-engaging element remains in contact with the tissue, responsively to the electrical signal, providing a first output indicative of a location of the anchor within the heart;
during driving of the tissue-engaging element into the tissue, continuing to receive the electrical signal as the electrical signal becomes indicative of a response of the tissue to the anchoring; and
responsively to the electrical signal indicative of the response of the tissue, providing a second output indicative of a position of the anchor within the heart.

There is further provided, in accordance with some applications, a data-processing apparatus including means for carrying out the steps of the method.

There is further provided, in accordance with some applications, a computer program including instructions which, when the program is executed by a computer, cause the computer to carry out the method.

There is further provided, in accordance with some applications, a computer-readable medium having stored thereon the computer program.

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. Various features and steps as described elsewhere in this disclosure may be included in the examples summarized here.

### BRIEF DESCRIPTION OF THE DRAWINGS

Figs. 1 and 2A-B are schematic illustrations of a system comprising an implant, a multi-component delivery tool, and a sensing device, in accordance with some applications of the present invention;
Figs. 3A-C are schematic illustrations of various outputs provided by a display of the sensing device of Figs. 1-2B;
Figs. 4A-D shows experimental data of electrical signals received by the sensing device of Figs. 1-2B;
Figs. 5A-5B are schematic illustrations of an exemplary system, in accordance with some applications;
Figs. 6A-B, 7A-B, 8, and 9A-9B are schematic illustrations of various systems, in accordance with some applications;
Fig. 10 shows experimental data received during a time period in which an anchor penetrates tissue of an annulus; and
. Figs. 11 and 12 are schematic illustrations of distal parts of delivery tools that are configured to facilitate determination of their orientation within the heart, in accordance with some applications.

### DETAILED DESCRIPTION

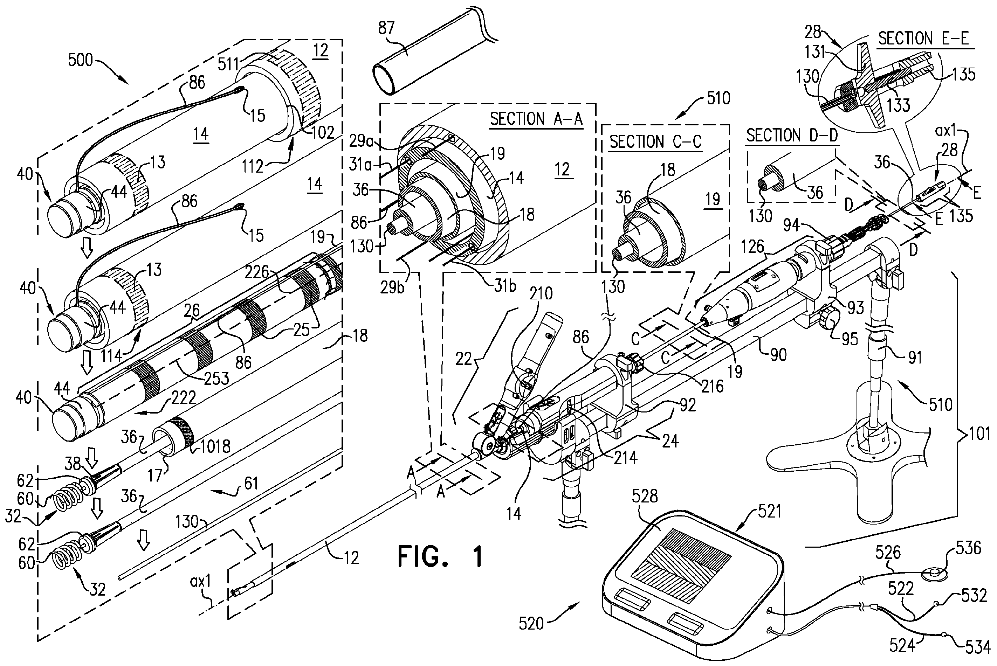

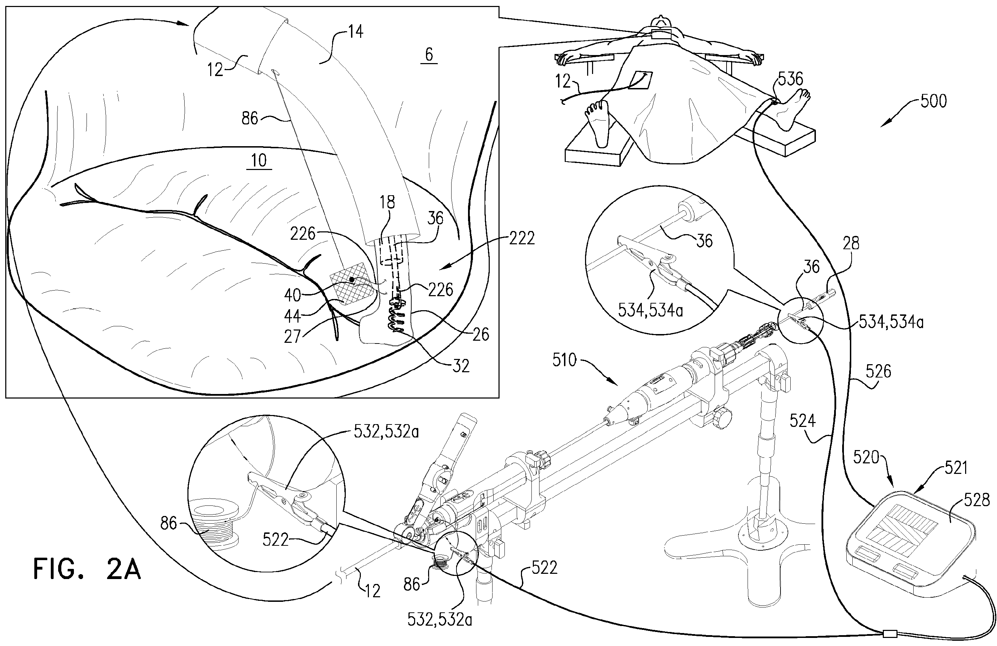

Reference is now made to Figs. 1, and 2A-B, which are schematic illustrations of a system 500 comprising an implant 222, a multi-component delivery tool 510, and a sensing (and/or procedure-aiding) device 520, in accordance with some applications of the present invention. Tool 510 comprises one or more rotationally-controlled steering catheters configured for delivering the implant to a heart of a subject. As described in detail hereinbelow, device 520 comprises a data-processing system 521 (e.g. a processor), which is configured to provide an indication of the location of an anchor of implant 222 within the heart, during implantation of the implant.

In the example shown, implant 222 is an annuloplasty implant, such as an annuloplasty ring structure (e.g. comprising a flexible sleeve 26 and a contracting mechanism 40), but it is to be noted that the techniques disclosed herein may be used for other implants, *mutatis mutandis.* Sleeve 26 may comprise a braided fabric mesh, e.g., comprising polyethylene terephthalate (such as Dacron (TM)). Implant 222 (e.g. sleeve 26 thereof) may be configured to be placed only partially around a cardiac valve annulus 10 (i.e., to assume a C-shape), or alternatively entirely around the valve annulus. Once anchored in place, implant 222 (e.g. sleeve 26 thereof) may be contracted so as to circumferentially tighten the valve annulus.

Sleeve 26 has (a) a tubular lateral wall 253 that (i) circumscribes a central longitudinal axis of the sleeve, and (ii) defines the lumen of the sleeve 26.

In order to tighten annulus 10, implant 222 comprises a flexible elongated contraction member 226 that extends along sleeve 26. Elongated contraction member 226 comprises a wire, a ribbon, a rope, or a band, which may comprise a flexible and/or superelastic material, e.g., nitinol, polyester, stainless steel, or cobalt chrome. For some applications, the wire comprises a radiopaque material. For some applications, contraction member 226 comprises a braided polyester suture (e.g., Ticron). For some applications, contraction member 226 is coated with polytetrafluoroethylene (PTFE). For some applications, contraction member 226 comprises a plurality of wires that are intertwined to form a rope structure.

Implant 222 may further comprise a contracting mechanism 40 (e.g. an adjustment mechanism), which facilitates contraction of the implant so as to facilitate adjusting of a perimeter of the annulus and leaflets of the cardiac valve. Contracting mechanism 40 comprises a rotatable structure (e.g., a spool, as described hereinbelow) that is disposed within a housing 44. For some applications of the present invention, contracting mechanism 40 comprises the housing 44. Contracting mechanism 40 may be surrounded by a braided mesh, coupled (e.g., by being sutured or otherwise coupled) to the braided mesh of sleeve 26. For some applications, and as shown, contracting mechanism 40 is coupled to an outer, lateral surface of sleeve 26.

For some applications, implant 222 (including contracting mechanism 40), and/or delivery tool 510 is as described in one or more of the following references, *mutatis mutandis:*
US Patent Application Publication 2014/0309661 to Sheps et al.
US Patent Application Publication 2015/0272734 to Sheps et al.
US Patent Application Publication 2018/0049875 to Iflah et al.

Fig. 1 shows the concentric relationship between components of delivery tool 510 (in an exploded view in the frame on the left-side of Fig. 1). Typically, delivery tool 510 comprises a first, outer catheter 12 comprising a sheath configured for transluminal advancement through vasculature of a subject. For some applications of the present invention, outer catheter 12 comprises a sheath configured for advancement through a femoral artery toward an atrium 6 of a heart of a subject. Tool 510 comprises a second catheter, or guide catheter 14, comprising a distal end portion 114 that is configured to pass through catheter 12 (i.e., a primary lumen thereof), to become disposed outside of a distal end 102 of the outer catheter 12, and to be oriented in a desired spatial orientation within the atrium.

Distal end portion 112 of outer catheter 12 is typically steerable. That is, distal end portion 112 is deflectable with respect to an immediately more proximal portion of catheter 12 (e.g., by using extracorporeal elements of tool 510). Distal end portion 112 may comprise a pull ring 511 that is coupled to two or more pull wires 29a and 29b, that are disposed within respective secondary lumens within a lateral wall of catheter 12 (as shown in section A-A of Fig. 1). As shown in the exploded view, guide catheter 14 is configured to be concentrically disposed within the lumen of catheter 12. Distal end portion 114 of guide catheter 14 is also typically steerable. That is, distal end portion 114 is deflectable with respect to an immediately more proximal portion of catheter 14 (e.g., by using extracorporeal elements of tool 510). Distal end portion 114 may comprise a pull ring 13 that is coupled to two or more pull wires 31a and 31b, that are disposed within respective secondary lumens within a wall of catheter 14 (as shown in section A-A of Fig. 1).

Guide catheter 14 is steerable to a desired spatial orientation in order to facilitate advancing and implantation of an implant in a body cavity of the subject, typically an atrium upstream of an atrioventricular valve such as the mitral valve or the tricuspid valve.

For applications in which tool 510 is used to deliver an implant to the mitral valve of the subject, typically, outer catheter 12 is configured for initial advancement through vasculature of the subject until a distal end 102 of catheter 12 is positioned in the left atrium. The distal steerable end portion of catheter 12 is then steered such that distal end 102 of catheter 12 is positioned in a desired spatial orientation within the left atrium.

For applications in which tool 510 is used to deliver an implant to the tricuspid valve of the subject, outer catheter 12 is typically configured for initial advancement through vasculature of the subject until a distal end 102 of catheter 12 is positioned in the right atrium. The distal steerable end portion of catheter 12 is then steered such that distal end 102 of catheter 12 is positioned in a desired spatial orientation within the right atrium.

The steering procedure is typically performed with the aid of imaging, such as fluoroscopy, transesophageal echo, and/or echocardiography. Following the steering of the distal end portion of catheter 12, guide catheter 14 (which houses implant 222) is advanced through catheter 12 in order to facilitate delivery and implantation of implant 222 along the annulus of the mitral valve. During the delivery, at least a portion of steerable distal end portion 114 is exposed from distal end 102 of catheter 12 and is thus free for steering toward the annulus of the mitral valve, as is described hereinbelow.

During delivery of sleeve 26 to the annulus of the cardiac valve, sleeve 26 and contracting mechanism 40 are disposed within a lumen of catheter 14 and are typically aligned longitudinally with a longitudinal axis of catheter 14. Mechanism 40 can be coupled to sleeve 26 in a manner that allows mechanism 40 to move (e.g., to translate) from a state in which it is in line with the longitudinal axis of catheter 14 (Fig. 1) to a state in which it is disposed alongside sleeve 26 (Figs. 2A-B). For example, contracting mechanism 40 may be coupled to sleeve 26 via one or more connectors 27, such as sutures, which provide flexible and/or articulated coupling. For some applications, the positioning of contracting mechanism 40 alongside a portion of sleeve 26 exposes a driving interface of the rotational structure (e.g., a driving interface), providing access to the interface for an adjustment tool 87 that is subsequently guided toward contracting mechanism 40 via a guide member 86.

A flexible, longitudinal guide member 86 (e.g., a wire) is coupled to a portion of contracting mechanism 40 (e.g., a portion of the rotatable structure, as described hereinbelow). Guide member 86 has a thickness of 0.35-0.45 mm, e.g., 0.4 mm. Guide member 86 is configured to facilitate guiding of adjustment tool 87 via guide member 86 and toward the rotatable structure of contracting mechanism 40. Typically, adjustment tool 87 is configured to engage the rotatable structure of contracting mechanism 40 following implantation of sleeve 26 along the annulus of the cardiac valve (e.g. as described, *mutatis mutandis*, in US 2014/0309661 to Sheps et al. and/or US 2015/0272734 to Sheps et al). Guide member 86 extends from contracting mechanism 40, alongside a portion of distal end portion 114 of guide catheter 14, and into a secondary lumen in the wall of guide catheter 14 via an opening 15 in guide catheter 14. Guide member 86 extends through the secondary lumen of guide catheter 14 (as shown in section A-A in Fig. 1) and has a proximal end that is accessible from outside the body of the subject. The secondary lumen in the wall of guide catheter 14 facilitates passage of guide member 86 through tool 510 without interfering with the other concentrically-disposed elongate tubular members that pass concentrically through the lumen of guide catheter 14.

In addition, system 500 comprises a plurality of anchors 32, typically between about 5 and about 20 anchors, such as about 10 or about 16 anchors. Each anchor 32 may comprise a tissue-coupling element 60 (e.g., a helical tissue-coupling element), and a tool-engaging head 62 (e.g., a non-helically-shaped portion), fixed to one end of the tissue-coupling element. Only one anchor 32 is shown in Figs. 1 and 2A-B as being reversibly coupled to a deployment element 38 of an anchor driver 36 of an anchor deployment manipulator 61. However, each of anchors 32 is reversibly couplable to a deployment element 38 of one or more anchor drivers 36. When sleeve 26 is disposed along the annulus of the cardiac valve, deployment manipulator 61 is configured to advance within a lumen of sleeve 26 and deploy each anchor 32 from within sleeve 26 through a wall of sleeve 26 and into cardiac tissue, thereby anchoring sleeve 26 around a portion of the valve annulus. The insertion of the anchors 32 into the sleeve and deployment of the anchors into cardiac tissue is described in detail hereinbelow.

Typically, but not necessarily, anchors 32 comprise a biocompatible material such as stainless steel 316 LVM. For some applications, anchors 32 comprise nitinol. For some applications, at least one part of each anchor 32 is coated with a non-conductive material.

Deployment manipulator 61 comprises anchor driver 36 and deployment element 38. For some applications, deployment manipulator 61 comprises channel 18.

Sleeve 26 is typically disposed within a lumen of guide catheter 14. Forces are applicable to a proximal end of sleeve 26 via a reference-force tube 19, a distal end of which is coupled to the proximal end of the sleeve. As shown, an implant-decoupling channel 18 is advanceable within a lumen of reference-force tube 19 and within a lumen of sleeve 26. As shown in the enlarged image of Fig. 1, a distal end 17 of implant-decoupling channel 18 is placeable in contact with an inner wall of sleeve 26, e.g., at a distal end thereof. The distal end portion of channel 18 may comprise a radiopaque marker 1018. As shown, tube 19 and sleeve 26 are longitudinally and coaxially disposed with respect to each other.

For some applications, channel 18 is steerable.

Typically, manipulator 61 advances within channel 18. For some applications, tool 510 comprises a plurality of anchor drivers 36 of manipulator 61, each driver 36 being coupled to a respective anchor 32. Each driver 36 is advanced within channel 18 in order to advance and implant anchor 32 in tissue. Following implantation of anchor 32, anchor 32 is decoupled from driver 36, as described herein, and driver 36 is removed from within channel 18. A subsequent anchor 32 is then advanced within channel 18 while coupled to a driver 36 (e.g., a new driver).

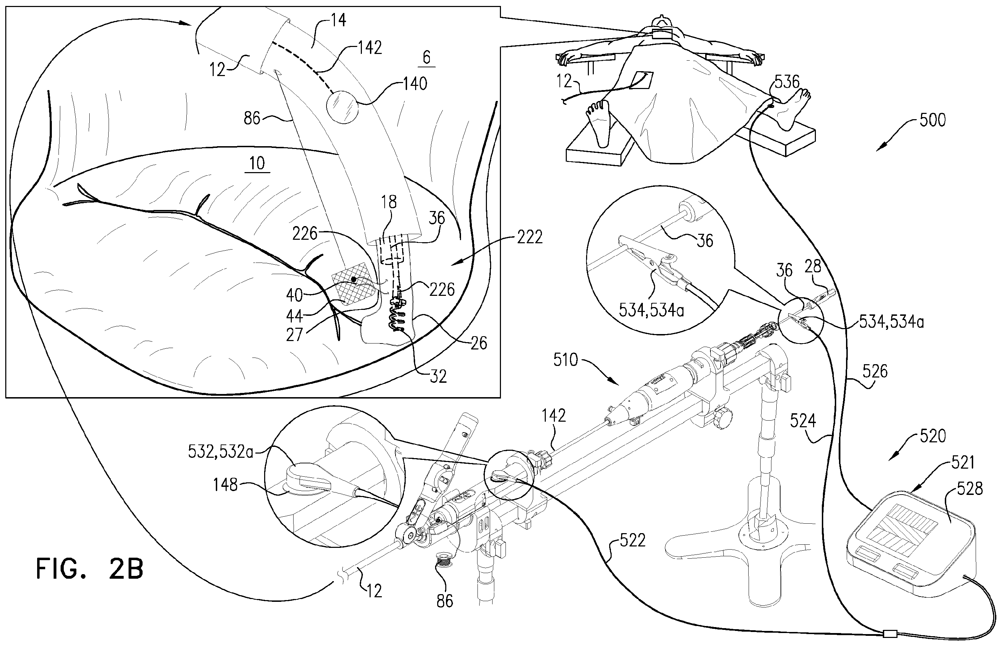

As will be described hereinbelow, and as shown in Figs. 2A and 2B, a first one of anchors 32 is configured to be deployed through sleeve 26 into cardiac tissue, when sleeve 26 is positioned along the annulus of the valve. The positioning and/or anchoring of the anchors 32 within the heart may be facilitated by device(s) 520, and/or 2520, and/or 3520, as will be explained in more detail hereinbelow. Following the deployment of the first anchor, a distal portion of sleeve 26 is slid distally off a portion of implant-decoupling channel 18. In order to decouple sleeve 26 distally from a portion of outer surface of channel 18, (1) a proximal force is applied to channel 18, while (2) reference-force tube 19 is maintained in place in a manner in which a distal end of tube 19 provides a reference force to sleeve 26, thereby facilitating freeing of a successive portion of sleeve 26 from around channel 18. Channel 18 is then positioned at a successive location within the lumen of sleeve 26 while tube 19 and/or catheter 14 is steered toward a successive location along the annulus of the valve (as will be described hereinbelow). Consequently, the successive portion of sleeve 26 provides a free lumen for advancement of a successive anchor 32 and deployment of the anchor through the wall of the sleeve at the successive portion thereof. Such freeing of the successive portion of sleeve 26 creates a distance between successive anchors deployed from within the lumen of sleeve 26.

For some applications, sleeve 26 comprises a plurality of radiopaque markers 25, which are positioned along the sleeve at respective longitudinal sites. The markers 25 may provide an indication in a radiographic image (such as a fluoroscopy image) of how much of the sleeve 26 has been deployed at any given point during an implantation procedure, in order to enable setting a desired distance between anchors 32 along the sleeve 26. For some applications, the markers comprise a radiopaque ink.

Typically, at least some (e.g., at least three, such as all) of the longitudinal sites are longitudinally spaced at a constant interval. Typically, the longitudinal distance between the distal edges of adjacent/consecutive markers, and/or the distance between the proximal edges of adjacent markers, is set equal to the desired distance between adjacent anchors. For example, the markers 25 may comprise first, second, and third markers, which first and second markers are adjacent, and which second and third markers are adjacent, and the distance between the proximal and/or distal edges of the first and second markers equal the corresponding distance between the proximal and/or distal edges of the second and third markers. For example, the distance may be between 3 and 15 mm, such as 6 mm, and the longitudinal length of each marker may be between 0.1 and 14 mm, such as 2 mm. (If, for example, the distance was 6 mm and the lengths were 2 mm, the longitudinal gaps between adjacent markers would have lengths of 4 mm.)

Each anchor 32 is coupled to deployment element 38 of an anchor driver 36. Anchor driver 36 typically comprises an elongate and flexible shaft (which is typically tubular) having at least a flexible distal end portion. The elongate shaft of driver 36 extends within a lumen of channel 18, through tool 510 toward a proximal end of a proximal handle portion 101 of tool 510. The tube of anchor driver 36 provides a lumen for slidable advancement therethrough of an elongate rod 130. Rod 130 facilitates the locking and unlocking of anchor 32 to deployment element 38. As shown in Section E-E of Fig. 1, a proximal end of rod 130 is coupled to a component of an anchor-release mechanism 28 at a proximal end of tool 510. Mechanism 28 comprises a housing 135 and a finger-engager 131 that is coupled to the proximal end of rod 130. Finger-engager 131 is coupled to a housing 135 via a spring 133 (section E-E of Fig. 1). A proximal end of the tube of anchor driver 36 is coupled to housing 135. The physician releases anchor 32 from deployment element 38 when finger-engager 131 is pulled proximally, thereby retracting rod 130 proximally.

Proximal handle portion 101 is supported by a stand having support legs 91 and a handle-sliding track 90. Handle portion 101 comprises an outer-catheter handle 22, a guide-catheter handle 24, an implant-manipulating handle 126, and anchor-release mechanism 28. Handle 22 is coupled to a proximal end of outer catheter 12. Handle 24 is coupled to a proximal portion of guide catheter 14. Handle 126 is coupled to a proximal portion of reference-force tube 19, and linear movement of handle 126 with respect to handle 24 moves reference-force tube 19 (and thereby typically implant 222) through catheter 14. As described hereinabove, housing 135 of anchor-release mechanism 28 is coupled to a proximal portion of the tube of anchor driver 36. The relative positioning of each of the concentrically-disposed components of tool 510 is shown in the exploded view and sections A-A, C-C, and D-D of Fig. 1.

The stand supporting proximal handle portion 101 may be moved distally and proximally to control a position of the entire delivery tool 510, particularly so as to adjust a distance of distal end 102 of catheter 12 from the interatrial septum. Handle 22 comprises a steering knob 210 that is coupled to steering wires 29a and 29b disposed within respective secondary lumens in the wall of outer catheter 12. Rotation of knob 210 adjusts a degree of tension of wires 29a and 29b which, in turn, apply a force to pull ring 511 at the distal end portion of outer catheter 12. Such force steers the distal end portion of catheter 12 within the atrium of the heart of the subject in a manner in which the distal end portion of catheter 12 is steered in a first steering plane that is typically parallel with the plane of the annulus of the valve (e.g., in a direction from the interatrial septum toward surrounding walls of the atrium). For some applications of the present invention, the distal end portion of catheter 12 may be preshaped so as to point downward toward the valve. For some applications, the distal end portion of catheter 12 may be pulled to assume an orientation in which the distal end portion points downward toward the valve. For some applications of the present invention, the distal end portion of catheter 12 is not made to point downward toward the valve.

Handle 24 is coupled to track 90 via a first mount 92. Mount 92 is slidable proximally and distally along track 90 in order to control an axial position of guide catheter 14 with respect to outer catheter 12. Mount 92 is slidable via a control knob 216. For example, control knob 216 of mount 92 controls the proximal and distal axial movement of the distal steerable portion of guide catheter 14 with respect to distal end 102 of outer catheter 12. Handle 24 comprises a steering knob 214 that is coupled to steering wires 31a and 31b disposed within respective secondary lumens in the wall of guide catheter 14. Rotation of knob 214 adjusts a degree of tension of wires 31a and 31b which, in turn, apply a force to pull ring 13 at the distal end portion of guide catheter 14. Such force steers the distal end portion of catheter 14 in a second steering plane within the atrium of the heart of the subject, typically downward and toward the annulus of the cardiac valve. Typically, as described hereinbelow, the distal end portion of guide catheter 14 is steered in the second plane that is substantially perpendicular with respect to the first plane in which the distal end portion of outer catheter 12 is steered.

The combined steering of the respective distal end portions of catheters 12 and 14 directs sleeve 26 down toward the annulus (e.g., via the steering of the distal end portion of catheter 14) and along the perimeter of annulus (e.g., from the posterior section of the valve to the anterior section of the valve, and vice versa), via the steering of the distal end portion of catheter 12.

For some applications, handle 22 may be tilted by the operating physician, in order to further adjust a position of the distal end of catheter 12.

Handle 126 is slidably coupled to track 90 via a second mount 93. Mount 93 is slidable proximally and distally along track 90, in order to control an axial position of reference-force tube 19 and at least a proximal portion of sleeve 26 with respect to guide catheter 14. For some applications, mount 93 comprises a control knob 95. For some such applications, control knob reversibly locks mount 93 to track 90, thereby reversibly inhibiting sliding of the mount along the track. Alternatively or additionally, turning of control knob 95 may cause sliding of mount 93 along track 90 (e.g., acting like a rack and pinion). For some applications, friction between (i) reference-force tube 19 and (ii) catheter 14 and/or handle 24 reduces a likelihood of inadvertent sliding of tube 19 through catheter 14, and thereby obviates the need for locking of mount 93 to track 90. Taken together with the steering of the distal end portion of guide catheter 14, such movement of tube 19 and at least the proximal portion sleeve 26 moves the proximal portion of sleeve 26 toward a desired portion of tissue of the annulus of the valve during deployment of anchors 32 from within the lumen of sleeve 26, as is described hereinbelow.

As is described hereinabove, in order to decouple sleeve 26 from a portion of an outer surface of channel 18, (1) channel 18 is pulled proximally, while (2) reference-force tube 19 is maintained in place. A proximal end of channel 18 is coupled to a knob 94 which adjusts an axial position of channel 18 proximally and distally with respect to reference-force tube 19 and sleeve 26.

Typically, handle portion 101 comprises a release-decision-facilitation member, such as a latch or button, that automatically engages when a given length of sleeve 26 has advanced off channel 18 (e.g., when channel 18 is at a given position with respect to tube 19); typically, just before sleeve 26 becomes completely decoupled from channel 18.

Handle portion 101 (comprising handles 22, 24, and 126 and anchor-release mechanism 28) has a length L1 of between 65 and 85 cm, e.g., 76 cm. Typically, as shown, a majority of the body portion of outer-catheter handle 22 is disposed at a non-zero angle with respect to a longitudinal axis ax1 of the multiple components of tool 510. The steering mechanism provided by handle 22 in order to steer the distal end portion of catheter 12 is disposed within the portion of handle 22 that is disposed at the non-zero angle with respect to axis ax1. Handle 22 comprises an in-line tubular portion which is longitudinally disposed in-line along axis ax1 and coaxially with respect to handles 24 and 126 and release mechanism 28. The in-line tubular portion is shaped so as to define a lumen for inserting guide catheter 14 therethrough and subsequently into the lumen of outer catheter 12. The in-line tubular portion has a length L24 of between 7 and 11 cm, e.g., 7 cm. Such spatial orientation of the majority of handle 22 at an angle with respect to axis ax1 reduces an overall functional length of handle portion 101.

In order to intraoperatively determine whether an anchor 32 is optimally positioned for anchoring, prior to driving the anchor into the tissue, the anchor 32 is typically placed against the tissue at a potential anchoring site, and the anchor 32 serves as a sensing electrode, via which data-processing system 521, electrically connected to the anchor, can acquire an electrical signal produced by the heart. Based on the detected signal (hereinbelow referred to as "the first signal"), data-processing system 521 provides an indication of the location of the anchor 32 within the heart, which the operator (e.g., physician) can use to facilitate optimal anchoring of the anchor.

As shown in Figs. 2A and 2B, a first wire 524 extending from data-processing system 521 is mechanically and electrically connectable to a proximal section of anchor driver 36, in order to place the data-processing system in electrical connection with anchor 32. For example, and as is shown in Figs. 2A and 2B, a connector 534, at an end of wire 524, may be mechanically and electrically connected to an accessible part of an electrically conductive shaft of anchor driver 36 that is disposed outside of the subject. Figs. 2A and 2B show an application in which connector 534 is embodied as a crocodile clip 534a, which is clipped onto the shaft of driver 36. However, it is to be understood that the scope of the present disclosure includes the use of other suitable electrical and/or mechanical connector means, such as a discrete snap-fit or screw-on connector at the proximal part of anchor driver 36.

In applications in which rod 130 is used in order to facilitate the locking and unlocking of anchors 32 to deployment element 38, the rod may itself serve as the electrical connection between anchor 32 and connector 534 e.g., by the rod itself being electrically conductive and/or having an electrically conductive element extending therethrough or therealong. For such applications, electrical connection between rod 130 and connector 534 may be achieved via part of the rod being exposed at a proximal end of anchor driver 36 (e.g., through a window defined in the shaft of the anchor driver or in housing 135, or protruding proximally from the housing), or via part of the shaft or part of the housing of the anchor driver being conductive, and providing an electrical connection between the connector and the rod.

In this state, anchor 32 is thus usable as an electrode to detect electrophysiological signals produced by the heart, during implantation (e.g., of implant 222) within the heart.

As described hereinabove, anchor 32 is typically placed against the tissue at a potential anchoring site in order for data-processing system 521 to assess the suitability of the potential anchoring site within the heart for anchoring of the anchor. It is to be noted that, in this context, the term "placed against" may include (i) placing the anchor in direct contact with the surface of the tissue at the potential anchoring site, (ii) driving the anchor at least partway into the tissue, or (iii) sandwiching sleeve 26 between the anchor and the tissue (e.g., without direct contact between the anchor and the tissue).

For some applications, data-processing system 521 also receives an electrical signal (i.e., a second electrical signal) from an additional component of the implantation system within the heart (e.g. from delivery tool 510 and/or an additional component of implant 222 itself) - i.e. in addition to the signal from the anchor (which may be referred to as the first electrical signal). For example, this additional component may be a second sensing electrode (e.g., for sensing the electrophysiological signals produced by the heart), with the anchor being referred to as a first electrode. From the first and second signals, data-processing system 521 may derive a refined signal, which may be improved (e.g., to have a better signal-to-noise ratio) compared to the first signal alone. The refined signal may be used to provide an indication to the operator of the location of anchor 32 within the heart. Various applications are described hereinbelow for the second electrode, e.g., for some applications, an electrode 140 disposed at a distal part of catheter 14 serves as the second electrode (Fig. 2B), or, alternatively, for some applications, contracting member 40 serves as the second electrode (Fig. 2A), as will be explained in more detail hereinbelow. Typically, the second electrode is not in direct contact with the tissue during the detection of the signals - e.g., it may be suspended in the bloodstream, within the heart, and does not contact the tissue.

One or more reference electrodes 536, placed outside of the heart (e.g., on the skin of the subject, such as on a leg of the subject, as shown in Figs. 2A-B), may be connected to data-processing system 521 via a third wire 526, and may facilitate obtaining the first and/or the second signal. For example, the first signal may be a "trace" (e.g., an "ECG lead") between the first (anchor) electrode and reference electrode 536, the second signal may be a trace between the second electrode and the reference electrode, and the refined signal may be derived (e.g. by data-processing system 521) from the first signal and the second signal (e.g. by subtraction). It is to be understood that other analyses of the signals obtained via these three electrodes may also be used. A "skin-to-skin" signal, which may also be referred to as a "reference" signal, may further be used during the anchoring process. This reference signal may be obtained by placing two electrodes outside of the heart of the subject (e.g., a first reference electrode on the left arm of the subject, and a second reference electrode placed on the right leg of the subject).

For some applications, rather than data-processing system 521 receiving discrete first and second signals and deriving a refined signal therefrom, the data-processing system 521 may more directly receive the refined signal as a trace between the first electrode and the second electrode (e.g., a "bipolar signal"). For such applications, even though the refined signal is typically a product of the measured difference in electrical potential between these two electrodes, the electrical connection between data-processing system 521 and the first electrode may be considered to provide a "first signal" and the electrical connection between the data-processing system and the second electrode may be considered to provide a "second signal."

Data-processing system 521 may be adapted to, responsively to the first signal, the second signal, and/or a refined signal, determine the location of the anchor within the heart. That is, data-processing system 521 may be adapted to associate various signals with corresponding locations of anchor 32 within the heart (e.g., different tissues of the heart, or different locations along an atrioventricular axis of the heart). For example, a refined signal indicative of the anchor being placed against tissue of the annulus may be different than a refined signal indicative of the same anchor placed against tissue of atrium 6 or placed against tissue of the ventricle 8. If the data-processing system determines that the anchor is located satisfactorily within the heart, the anchor can then be driven into the tissue.

The refined signal which results from placing more than one electrode within the heart may advantageously improve the ability of data-processing system 521 to identify the location of anchor 32 within the heart, as compared with a signal that might be obtained by placing only a single electrode in the heart. For example, the refined signal may have an improved signal-to-noise ratio than an application in which only a single electrode is placed within the heart.

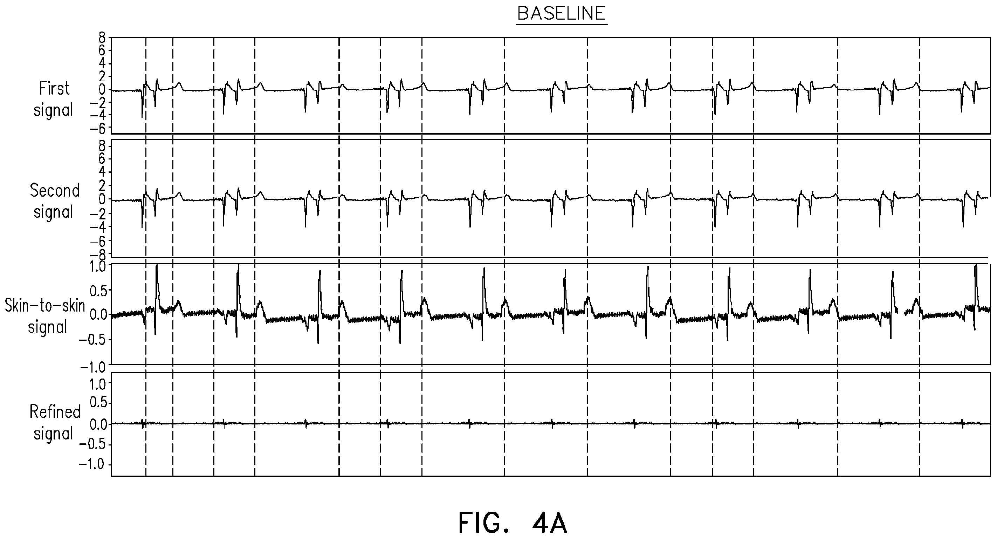

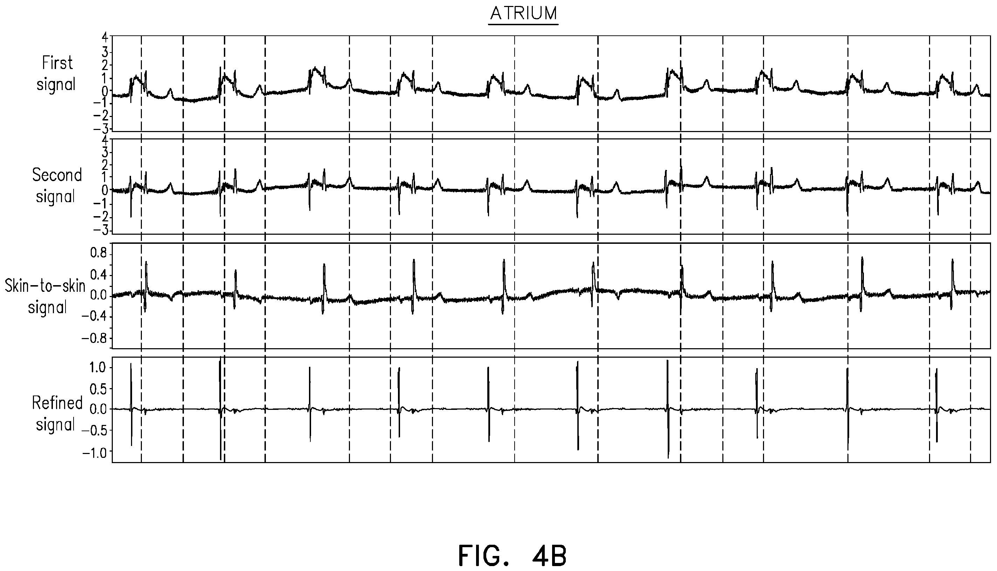

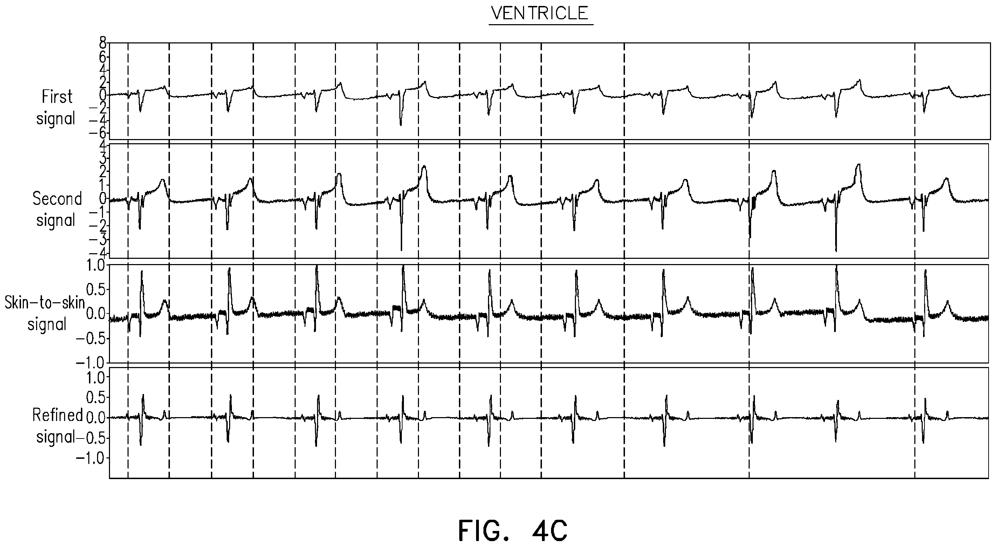

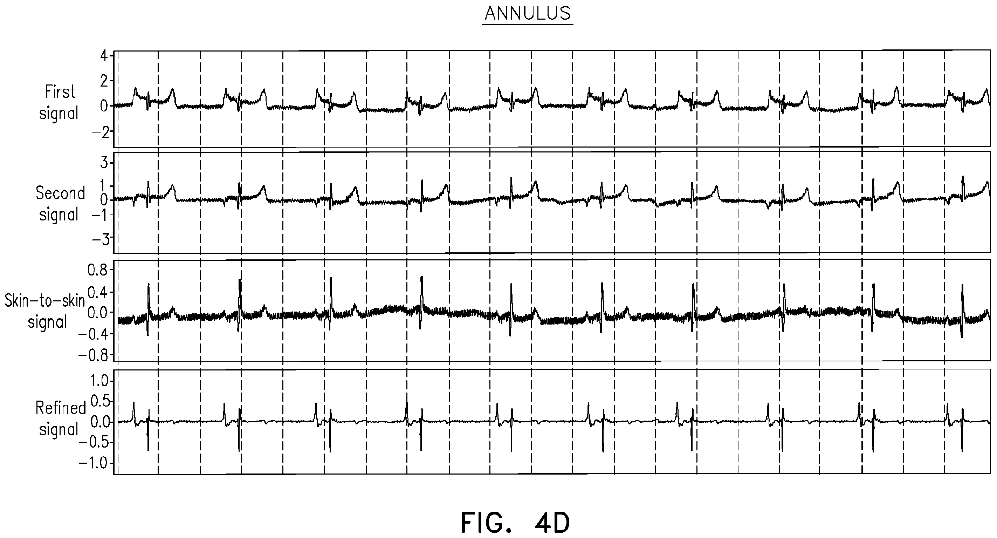

Figs. 4A-D show experimental data demonstrating that the refined signal more distinctly differs between locations of anchor 32 within the heart than does the first signal or the second signal alone. For example, in the experimental data, the refined signal was graphically distinct between the anchor contacting tissue of the atrium (Fig. 4B), tissue of the ventricle (Fig. 4C), and tissue of the annulus (Fig. 4D).

Prior to anchor 32 contacting the tissue, the baseline of the refined signal may be approximately zero (Fig. 4A), since both the anchor and the second electrode are suspended within the bloodstream of the heart, and are thus detecting substantially the same electrophysiological signals produced by the heart (e.g., the first and second signal cancel each other out). Thus, compared to a system in which only a single detecting electrode is used (e.g. the first signal alone), a change in the refined signal upon the anchor contacting the tissue is more pronounced (compare, for example, Fig. 4A to Figs. 4B-D). This change may advantageously provide the operator with an indication that anchor 32 has contacted tissue. Additionally, for some applications, receiving a refined signal of approximately zero despite contact between anchor 32 and the tissue being verified by other means (e.g., via imaging) may advantageously indicate that the second electrode is (e.g. undesirably) in contact with the tissue.

Typically, device 520 (e.g. data-processing system 521 thereof) runs a program in which at least one of the first, second, and refined signals serves as an input, and which responsively determines the location of anchor 32 within the heart. As described hereinabove, the program may receive the refined signal directly, or may derive the refined signal from the first and second signals (e.g., by subtraction). For some applications, the program determines the location of anchor 32 responsively to the refined signal alone. For some applications, the program determines the location of anchor 32 responsively to both the refined signal and the first signal. For some applications, the program determines the location of anchor 32 responsively to both the refined signal and the second signal.

For some applications, by running the program, data-processing system 521 determines a location of anchor 32 along the atrioventricular axis of the heart. For some such applications, data-processing system 521 determines whether anchor 32 is disposed in a predefined discrete position (which may be one of multiple predefined discrete positions), such as within the atrium, at the annulus, or within the ventricle. Additionally or alternatively, the data-processing system may determine whether the anchor is in contact with a discrete type of tissue, such as atrial wall tissue, annulus tissue, leaflet tissue, or ventricular wall tissue.

For some applications, artificial intelligence and/or machine learning is employed in the building of the program. For example, building of the program may be facilitated by the artificial intelligence analyzing data (e.g., a labeled data set) that may include (e.g. refined signals, first signals, second signal, and/or other ECG data obtained from previously performed procedures). For some applications, in order to train (e.g., further train) the program, the location outputted intra-procedurally by the program is compared to the actual location of the anchor (e.g., determined by other means).

Experiments have indicated that, for at least some applications in which the program is built and/or trained using artificial intelligence and/or machine learning, determining the location responsively to both the refined signal and the first signal (e.g., using both signals as inputs) can provide a more accurate determination of the location of anchor 32, compared to using either the first signal or the refined signal as an input without the other.

Device 520 typically comprises a display 528, for providing the operator with an output indicative of the location of anchor 32 that has been determined by the program run by data-processing system 521. For example, the output may be indicative of a location along the atrioventricular axis of the heart.

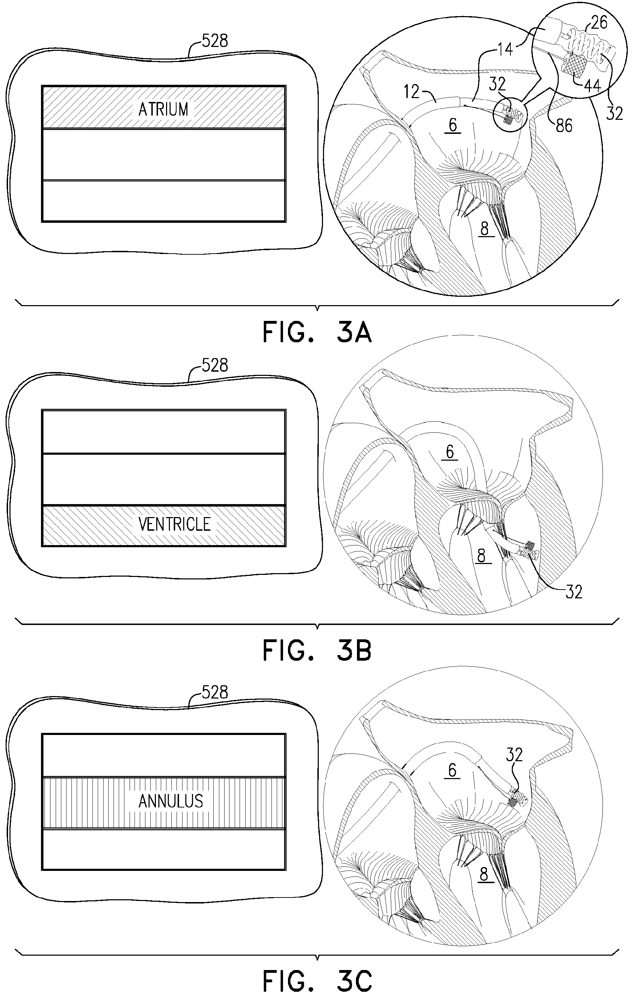

For some applications, and as illustrated in Figs. 3A-C, display 528 may provide the output as an indication of a discrete position of an anchor 32. For example, display 528 may indicate that the anchor is contacting tissue of the atrium (Fig. 3A), tissue of the ventricle (Fig. 3B), or tissue of the annulus (Fig. 3C).

For some applications, device 520 is configured to provide the output (e.g., on display 528) as a binary output (e.g., a yes/no or go/no-go output), responsively to data-processing system 521 determining that anchor 32 can be driven into the tissue at a potential anchoring site (e.g. by determining that the anchor is contacting tissue of the annulus).

For some applications, device 520 is not used as a primary navigation device, but instead is used to augment navigation/guidance provided by other means, such as imaging (e.g., fluoroscopy and/or ultrasound). For example, device 520 may be used to provide verification of the location of an anchor that has been determined via imaging. For example, the operator may perform an implantation procedure guided primarily by imaging but may drive the anchor into tissue only upon observing an output indicative of the anchor being in contact with tissue of the annulus, or only upon an absence of a warning that the anchor is in contact with tissue that is not of the annulus.

For some applications, the operator may reposition anchor 32 within the heart responsively to the output presented by display 528. For example, Figs. 3A-C may represent a series of steps performed by the operator, during implantation of implant 222 within the heart, in which an anchor 32 is initially incorrectly placed against tissue of the atrium (Fig. 3A), is subsequently incorrectly placed against tissue of the ventricle (Fig. 3B) and is finally correctly placed against tissue of the annulus (Fig. 3C).

For some applications, data-processing system 521 is supplied with a surgical plan, and is used to provide the operator with an indication of whether the anchor is positioned within the heart according to the surgical plan, responsively to the refined signal.

For some applications, display 528 alternatively or additionally presents a graphical representation of at least one of the first signal, the second signal, and the refined signal (e.g., as illustrated in Figs. 4A-D), to which the operator may refer.

Alternatively or additionally to using display 528 to provide a visual output, device 520 may provide its output via a different medium, such as an audio or tactile/haptic output.

Fig. 2A illustrates an exemplary method of using system 500, in which contracting mechanism 40 serves as the second electrode within the heart of the subject. In addition to first wire 524 which is connectable to anchor driver 36, a second wire 522 extends from data-processing system 521, and is mechanically and electrically connectable to a proximal section of guide member 86, in order to place the data-processing system in electrical connection with contracting mechanism 40. For example, and as shown in in Fig. 2A, a connector 532, at an end of wire 522, may be mechanically and electrically connected to an accessible part of guide member 86. Fig. 2A shows an application in which connector 532 is embodied as a crocodile clip 532a, which is clipped onto an extracorporeal section of guide member 86. In such an application, guide member 86 is typically electrically conductive (e.g. comprises an electrically conductive material such as a metal).

Fig. 2B illustrates a variant of system 500 in which, rather than contracting mechanism 40 serving as the second electrode, an additional electrode 140, disposed at a distal part of delivery tool 510, serves as the second electrode. Fig. 2B illustrates one such application, in which electrode 140 is exposed at the distal part of catheter 14. Electrode 140 may be embedded in, or fixed onto, the outside wall of the distal part of catheter. A wire (or other conductor) 142 extends, from electrode 140, proximally through catheter 14 (e.g., through the wall of the catheter, or within a lumen of the catheter), to an extracorporeal proximal portion (e.g. a handle) of the catheter, where connector 532 can be connected to the wire. Fig. 2B shows an application in which wire 142 terminates at an electronic snap 148, to which connector 532 (also embodied as an electronic snap 532b) is mechanically and electrically connected via a snap fit. It is to be understood that second electrode 140 may be disposed at a distal part of any of the components of delivery tool 510 and/or of implant 222, for example second electrode 140 may be disposed at a distal part of channel 18, on flexible sleeve 26, or on catheter 12 of the delivery tool.

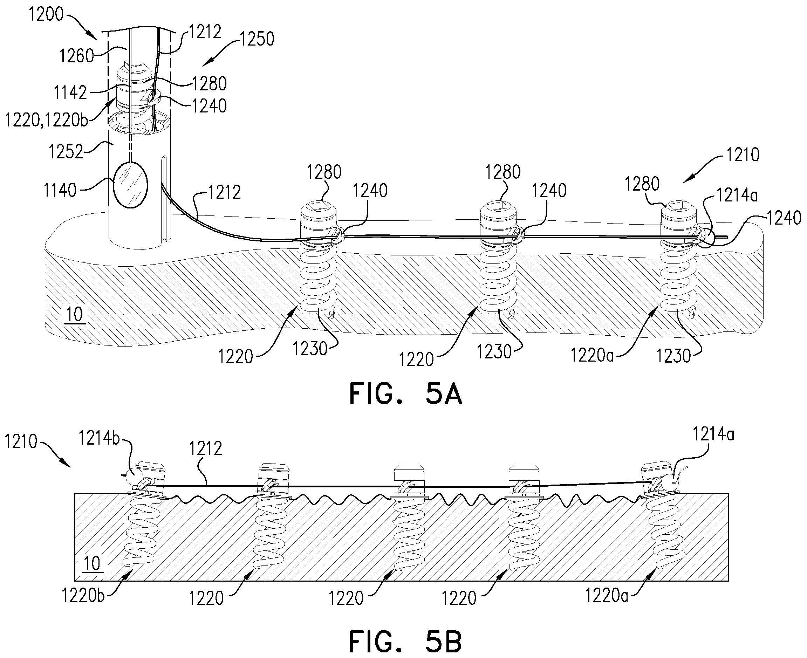

Figs. 5A-5B are schematic illustrations of an exemplary system 1200, in accordance with some applications. System 1200 comprises an implant 1210, a delivery tool 1250 for percutaneous implantation of the implant, and device 520 (or a variant of device 520) - although for simplicity device 520 is not shown in Figs. 5A-B. For some applications, system 1200 may be considered to be a variant of system 500.

Implant 1210 is typically an annuloplasty implant, and comprises a plurality of anchors 1220, and a contraction member 1212 (e.g., a tether). Each anchor 1220 typically comprises an anchor head 1280 having an eyelet 1240 through which contraction member 1212 is threaded, and a tissue-engaging element 1230 configured to be driven into tissue of annulus 10.

In contrast to implant 222 of system 500, implant 1210 does not comprise a sleeve (e.g., sleeve 26). Rather, in order to contract the annulus and reduce valve regurgitation, the anchors are typically driven into tissue of the annulus while threaded onto contraction member 1212, and the contracting member is subsequently tensioned in order to reduce the circumference of the annulus, thereby reducing valve regurgitation.

As illustrated in Figs. 5A, a distal part of delivery tool 1250 is typically advanced to the heart of the subject using a transluminal (e.g., transfemoral) approach, and anchors 1220 are subsequently anchored around the annulus of a valve of the heart. The anchors (e.g., eyelets 1240) are typically threaded onto the contraction member prior to their advancement, such that implanting the anchors around the annulus positions the contraction member around the annulus. Tension is subsequently applied to the contraction member, in order to contract the annulus, thus reducing valve regurgitation (Fig. 5B). In order to maintain the tension within the contraction member, a first locking bead 1214a is typically advanced to implant 1210 along with, and distally to, first anchor 1220a, along contraction member 1212, and a second locking bead 1214b is typically advanced to the implant along contraction member 1212 subsequently to anchoring the final tissue anchor 1220b to the annulus (Fig. 5B).

For some applications, implant 1210 and/or delivery tool 1250 is as described in one or more of the following references, *mutatis mutandis:*

US Patent Application Publication 2021/0145584 to Kasher et al.

US Provisional Patent Application 63/162,443 to Shafigh et al., filed March 17, 2021

International Patent Application PCT/IB2021/058665 to Halabi et al., filed September 23, 2021.

Similarly to the method described hereinabove with reference to system 500, prior to driving each anchor 1220 into the tissue at a potential anchoring site, device 520 is used to identify whether the potential anchoring site is suitable (e.g., whether the potential anchoring site is indeed on the annulus). As in system 500, the anchor is used as a first electrode, and an anchor driver 1260, which is used to drive the anchor into the tissue, may also be used to electrically connect data-processing system 521 (not shown) to the anchor. Similarly to the application shown in Fig. 2B, a second electrode 1140 disposed on a distal part of delivery tool 1250 (e.g. on a distal part of a delivery catheter 1252), may be connected to data-processing system 521 via a wire 1142 extending from the electrode, proximally along catheter 1252 (e.g. extending within the wall of the catheter, or within a lumen of the catheter as shown in Fig. 5A), to an extracorporeal proximal portion (e.g. a handle) of the catheter, where connector 532 can be connected to the wire, as described hereinabove with reference to Fig. 2B, *mutatis mutandis.*

For some applications in which contracting member 1212 is electrically conductive, it may be advantageous to electrically isolate anchors 1220 from the contraction member - e.g., in order to reduce electrical signals from being conducted (e.g. from previously-anchored anchors), along the contracting member to the anchor that is presently engaged by the driver, via which device 520 is electrically connected to the anchor that is presently engaged by the driver. For example, eyelets 1240 may comprise and/or be coated with an electrically-insulating material. However, it has been determined that data-processing system 521 is typically capable of determining the position of the presently-engaged anchor even in the absence of such insulation.

During anchoring of an anchor 1220, the known (e.g. fixed) distance between second electrode 1140 and the respective anchor may advantageously assist in the derivation of the refined signal. For example, data-processing system 521 may not need to recalibrate between anchoring of the anchors, as the distance between the second electrode and each anchor being anchored is fixed. It is to be understood that the applications described hereinabove with reference to Fig. 2B may provide a similar advantage.

Reference is again made to Figs. 1-5B. It is to be noted that, due to the mechanical disconnection of driver 36 from the anchor subsequently to anchoring, data-processing system 521 is typically electrically disconnected from the anchor. Thus, post-implantation, although the anchor remains in the heart, it ceases to serve as an electrode. In applications in which rod 130 provides driver 36 with its electrical conductivity, the electrical disconnection may occur upon retraction of the rod to release the anchor from the driver.

In addition, the second electrode may also be electrically disconnected from data-processing system 521 following implantation of implant 222. For example, in applications in which contracting mechanism 40 serves as the second electrode (e.g., as shown in Fig. 2A), guide member 86 may be disconnected from the contracting mechanism following actuation of the contracting mechanism.

For applications for which the second electrode is disposed at a distal part of delivery tool 510 (e.g., second electrode 140 as shown in Fig. 2B and second electrode 1140 as shown in Fig. 5A), the withdrawal of the delivery tool from the heart following implantation removes the second electrode from the heart.

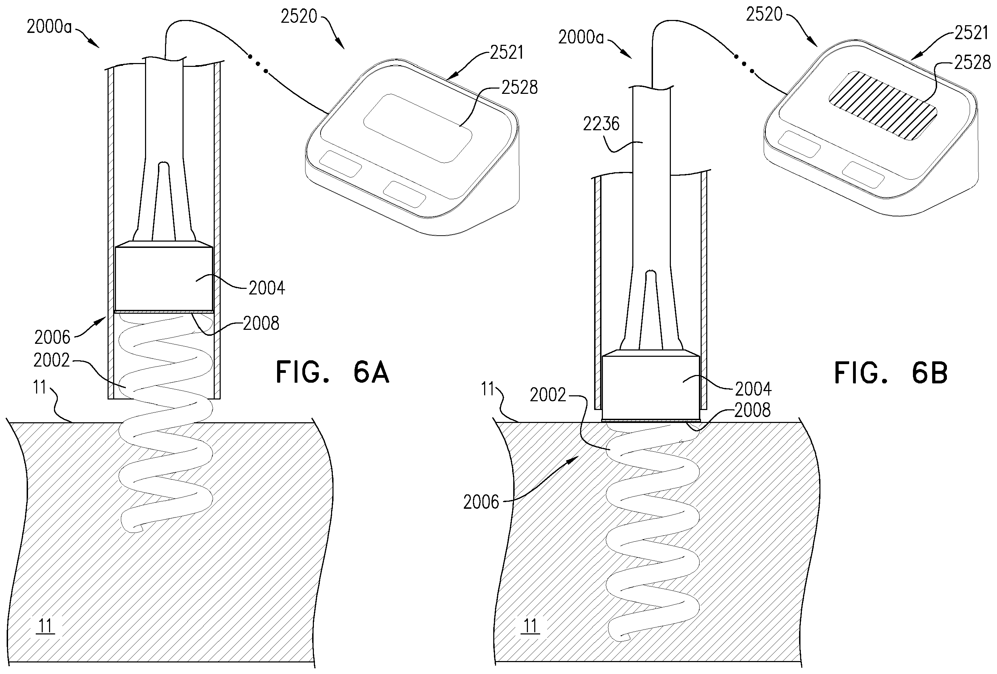

Reference is now made to Figs. 6A-B, 7A-B, 8, and 9A-9B, which are schematic illustrations of various systems, in accordance with some applications. These systems are configured to provide an indication that anchoring of an anchor has been successful and/or is complete (e.g., that the anchor has been driven into the tissue to the required depth), by determining that the anchor-head of the anchor has come into direct contact with the surface of tissue 11 to which the anchor is being anchored. Each of these systems comprises an anchor, an anchor driver, and a sensing device that comprises a data-processing system (e.g. a processor). Tissue 11 may be tissue of annulus 10, but may alternatively be another cardiac tissue, or a non-cardiac tissue of the subject.

In order to determine direct contact between the anchor-head and the tissue surface, a tissue-facing electrode disposed at the tissue-facing surface of the anchor-head serves as a detecting electrode, from which the data-processing system of the sensing device receives an electrical signal, e.g., an ECG signal. Once the tissue-facing surface of the anchor-head comes into direct contact with the tissue surface, the sensing device outputs an indication of this contact in response to the received electrical signal. Typically, the electrical signal is conducted to the sensing device via the anchor driver, e.g., as described hereinabove, *mutatis mutandis.*

For some applications, the electrical signal detected by the tissue-facing electrode is simply the electrophysiological signal produced by the heart, such that the tissue-facing electrode serves as a sensing electrode to detect electrical activity produced by the heart. Additionally or alternatively, an exogenous electrical signal may be provided for this purpose (e.g., via a reference electrode placed outside of the heart of the subject).

The anchor comprises a tissue-engaging element that is adapted to be driven into the tissue, and an anchor-head that has a tissue-facing surface that, once the tissue-engaging element has been fully driven into the tissue, contacts the tissue surface. The anchor is typically delivered to the heart and driven into the tissue using the anchor driver that is engaged with the anchor-head, e.g., as described hereinabove, *mutatis mutandis.*

Typically, the data-processing system runs a program in which the electrical signal obtained by the tissue-facing electrode serves as an input, and which responsively determines whether there is contact between the anchor-head and the tissue surface. For some applications, artificial intelligence and/or machine learning is employed in the building of the program. For example, building of the program may be facilitated by the artificial intelligence analyzing data (e.g., a labeled data set) of electrical signals (e.g. such electrical signals and/or other ECG data obtained from previously performed procedures). For some applications, in order to train (e.g., further train) the program, the location outputted intra-procedurally by the program is compared to the actual position of the anchor with respect to the tissue surface (e.g. determined by other means).

For some applications, determining contact between the anchor-head and the tissue may additionally or alternatively be used to determine whether the anchor has been driven into the tissue at an undesirable angle (e.g., oblique with the surface of tissue 11), rather than at an angle substantially perpendicular to the tissue (e.g. such that the anchor-head is generally parallel with the tissue).

For some applications, the data-processing system is adapted to determine whether the angle of attack falls within a predetermined range of angles-of-attack, and to responsively provide an output (e.g., an alert), should the determination be indicative of the angle of attack falling outside the predetermined range. Thus for some applications, the data-processing system only alerts the operator should the angle-of-attack be outside the predetermined range.

For some applications, the data-processing system is adapted to provide such an indication of anchor-driving angle - e.g., by determining whether the anchor head lies parallel with the tissue. For some such applications, the anchor may have multiple tissue-facing electrodes disposed along the tissue-facing surface of the anchor-head in a manner which facilitates such functionality. For example, receiving an electrical signal from only a subset of the tissue-facing electrodes may be indicative that the anchor extends into the tissue at an angle oblique to the surface of the tissue, and therefore only part of the tissue-facing surface of the anchor head is in contact with the tissue. Similarly, receiving an electrical signal from all of the tissue-facing electrodes may be indicative that the anchor extends into the tissue substantially orthogonally to the surface of the tissue, and therefore substantially all of the tissue-facing surface is in contact with the tissue.

For some applications, an amount by which the anchor has been driven into the tissue is determined and compared to the data-processing system's determination and/or indication of contact between the anchor head and the tissue surface. For example, should it be determined that contact has been made between the anchor-head and the tissue-surface earlier than would be expected from the amount by which the anchor has been driven into the tissue (e.g. for an anchor that has a helical tissue-engaging element, as would be expected from the number of rotations of the anchor), this may be indicative that the anchor has been driven into the tissue at an undesirably shallow angle.

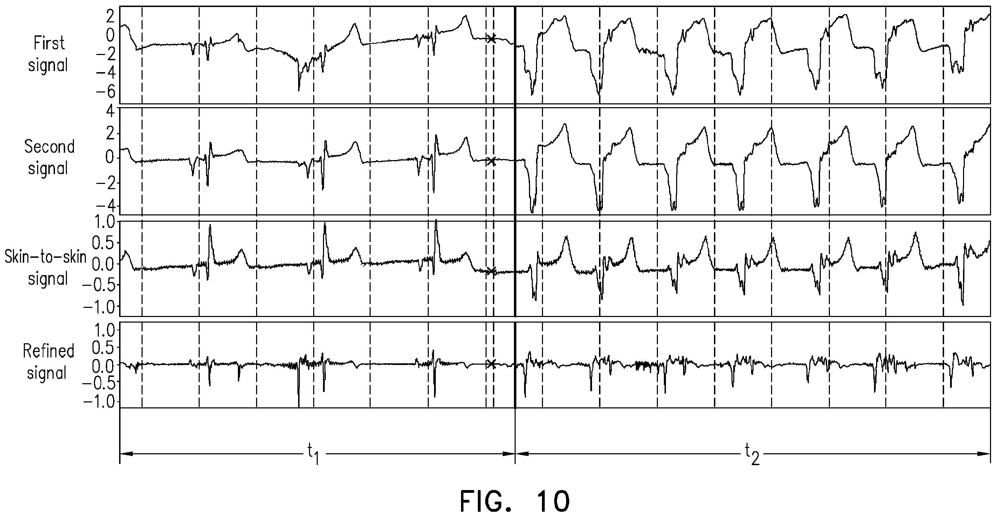

With regard to the previous paragraph, the amount by which the anchor has been driven into the tissue may be determined simply by the operator - e.g., counting rotations of the anchor driver, or observing fluoroscopically. Alternatively or additionally, the amount by which the anchor has been driven into the tissue may be determined by the data-processing system - e.g., responsively to receiving signals indicative of a response of the tissue to the driving of the tissue-engaging element into the tissue, e.g. as described hereinbelow, such as with reference to Fig. 10.

For some applications, the anchor driver is lockable to the anchor-head in a manner that places the tissue-facing electrode in electrical communication with the data-processing system without placing the tissue-engaging element in electrical communication with the data-processing system (i.e. such that the tissue-engaging element is electrically isolated from the data-processing system) - e.g. such that contact between the anchor-head and the tissue is distinguishable from contact between the tissue-engaging element and the tissue. Various applications for achieving the electrical isolation of the tissue engaging element from the data-processing system are described with respect to Figs. 6A-9B hereinbelow.

Figs. 6A-B show a system 2000a comprising an anchor 2006 that comprises a tissue-engaging element 2002, and an anchor-head 2004. System 2000a also comprises a sensing device 2520, which comprises a data-processing system 2521 (e.g. a processor), and a display 2528. For some applications, sensing device 2520 may be considered to be a variant of sensing device 520.

Anchor-head 2004 has a tissue-facing surface 2008 that may itself serve as the tissue-facing electrode. For example, anchor driver 2236 may be in electrical communication with tissue-facing surface 2008, such that placing the tissue-facing surface against the surface of tissue 11 provides an electrical communication between the tissue and data-processing system 2521. For some such applications, the entire anchor-head 2004 may be electrically conductive (e.g., constructed from a metallic material and/or coated with an electrically conductive coating), such that the anchor-head serves as the conductor between tissue-facing surface 2008 and the anchor driver 2236, via which the electrical signal is relayed to data-processing system 2521. Thus, for some applications, tissue-facing surface 2008 may not be distinct from other parts of the anchor-head.

For some applications, tissue-engaging element 2002 may be electrically nonconductive, such that only upon anchor-head 2004 contacting tissue 11 (e.g., only upon the tissue-facing electrode contacting the tissue), is data-processing system 2521 in electrical contact with the tissue. For example, tissue-engaging element 2002 may be formed from an insulating material, and/or may be coated in an insulating material.

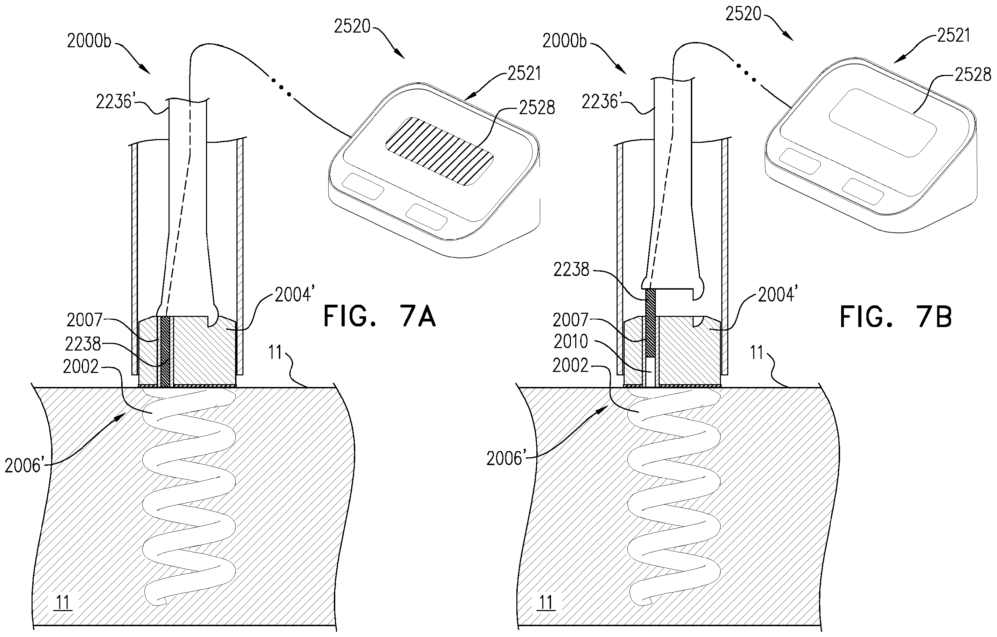

Figs. 7A-B show a system 2000b comprising an anchor 2006' that comprises tissue-engaging element 2002 and an anchor-head 2004'. System 2000b also comprises sensing device 2520, or a variant thereof, *mutatis mutandis.* Anchor 2006' is typically advanced and anchored using an anchor driver 2236', which may be considered to be a variant of the anchor drivers described hereinabove. Anchor 2006' and driver 2236' facilitate an alternative technique for providing electrical isolation between tissue-engaging element 2002 and the tissue-facing electrode. In such an application, the tissue-facing electrode is an electrode 2238 that is a component of anchor driver 2236', and extends distally (e.g., through an aperture 2010 in anchor-head 2004') to the tissue-facing surface of the anchor head, such that placing the anchor-head into contact with tissue 11 also places electrode 2238 into contact with the tissue. For some applications, aperture 2010 is lined with an insulating material 2007, such that electrode 2238 is electrically insulated from anchor 2006'. Thus, anchor driver 2236' is adapted to place data-processing system 2521 in electrical communication with tissue 11, via electrode 2238, without placing tissue-engaging element 2002 in electrical communication with the data-processing system.

Fig. 7A shows electrode 2238 in contact with tissue 11, such that display 2528 indicates contact between tissue-facing surface 2008 and tissue 11, providing the operator with feedback that the anchor has been fully implanted within the tissue (e.g., that tissue-engaging element 2002 is fully embedded within the tissue). Anchor driver 2236' can thus be disengaged from anchor 2006 and withdrawn, as illustrated in Fig. 7B, leaving the anchor implanted within the tissue.

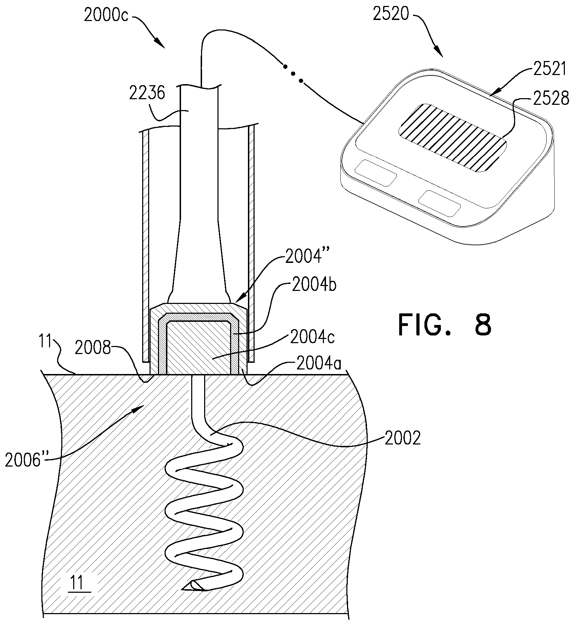

Fig. 8 shows a system 2000c comprising an anchor 2006" that comprises tissue-engaging element 2002 and an anchor-head 2004". System 2000c also comprises sensing device 2520, or a variant thereof, *mutatis mutandis.* Anchor 2006" is typically advanced and anchored using an anchor driver 2236, which may be considered to be a variant of the anchor drivers described hereinabove. Anchor 2006" and driver 2236 facilitate an alternative technique for providing electrical isolation between tissue-engaging element 2002 and the tissue-facing electrode. In such an application, anchor driver 2236 is coupled to an outer layer 2004a of anchor-head 2004", the outer layer being electrically conductive and extending to tissue-facing surface 2008 of anchor-head 2004". Outer layer 2004a serves as (or is electrically connected to) the tissue-facing electrode, such that the electrical signal is received by data-processing system 2521 via the electrical communication between the tissue-facing surface of the outer layer and the anchor driver.

As illustrated in Fig. 8, an insulating layer 2004b is provided to electrically isolate an inner core 2004c of anchor-head 2004" from the outer layer 2004a. Core 2004c is the part of anchor-head 2004" to which tissue-engaging element 2002 is coupled. Thus, data-processing system 2521 receives the electrical signal only from tissue-facing surface 2008 (i.e., not from the tissue-engaging element). Insulating layer 2004b may facilitate manufacturing of inner core 2004c and tissue-engaging element 2002 from an electrically conductive material (e.g., a metal), since any electrical signals to which these components are exposed would not interfere with the electrical signals conducted by outer layer 2004a. However, for some applications, rather than having a discrete insulating layer and a discrete inner core, the anchor-head may be manufactured primarily from an insulating material, but with electrically conductive outer layer 2004a.

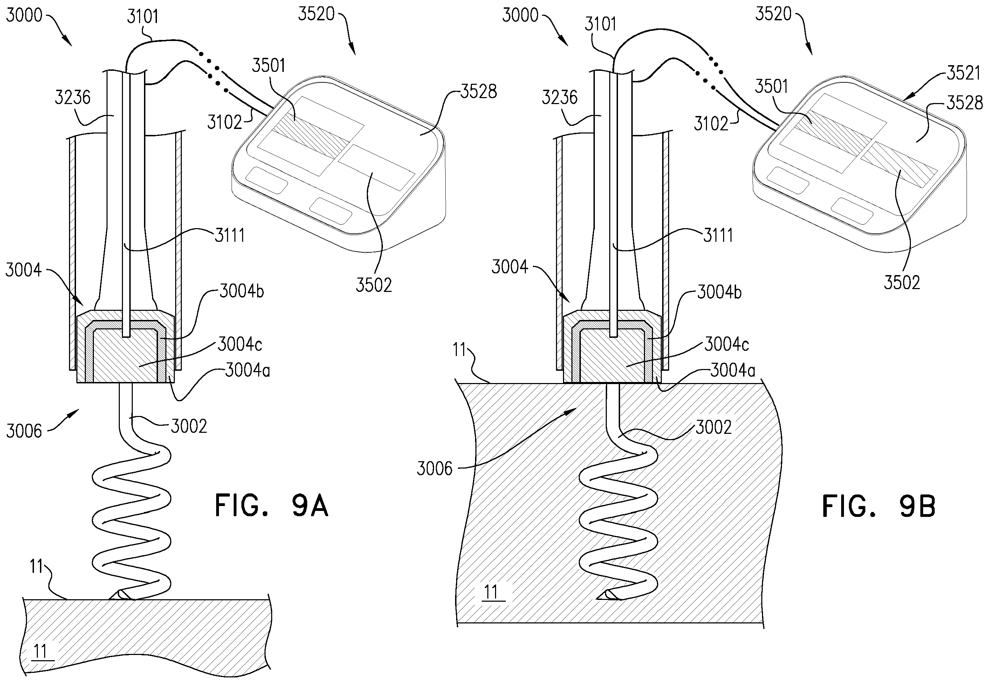

Figs. 9A-B show a schematic illustration of a system 3000 that comprises a sensing device 3520, for use with an anchor 3006. For some applications, sensing device 3520 may be considered to be a variant of sensing device 2520 and/or sensing device 520, *mutatis mutandis.* As shown in Figs. 9A-B, for some applications, anchor 3006 can be similar (e.g., substantially identical) to anchor 2006" (Fig. 8), except where indicated. For some applications, anchor 3006 can be similar (e.g., substantially identical) to anchor 2006' illustrated in Figs. 7A-B, for example, an electrode 2238 may extend through an insulated aperture of the anchor. Anchor 3006 is typically advanced and anchored using an anchor driver 3236, which may be considered to be a variant of the anchor drivers described hereinabove.

Device 3520 typically comprises a data-processing system 3521 (e.g. a processor), which may combine the functionality of data-processing systems 521 and 2521, such that it is adapted to both (i) provide, at least during a first phase of the anchoring process of anchor 3006 (Fig. 9A), an indication 3501 of the location of an anchor within the heart, and (ii) provide, at least during a second phase of the anchoring process (Fig. 9B), an indication 3502 of whether anchoring of an anchor has been successful and/or is complete (e.g. that the anchor has been driven into the tissue to the required depth). Device 3520 may be adapted to provide this indication via a display 3528, which may be considered a variant of, or a combination of, display 528 and/or display 2528.

In such applications, data-processing system 3521 is electrically connected (i) to a tissue-engaging element 3002 of the anchor, which serves as an electrode used for locating the anchor, as described with reference to Figs. 1-5B hereinabove (e.g. the "first electrode" in these figures), via a first electrical connection 3101, and additionally (ii) to a tissue-facing electrode 3004a of the anchor, which serves as an electrode used for determining contact between the anchor-head and the tissue, as described with reference to Fig. 8 hereinabove, via a second electrical connection 3102. In the example shown, electrode 3004a is as described hereinabove for electrode 2004a, *mutatis mutandis,* but may alternatively be as described hereinabove for electrode 2238, *mutatis mutandis.*

Thus, for such applications, tissue-engaging element 3002 serves as a "locator-electrode," and the electrode at the tissue-facing surface of the anchor-head serves as a "head-contact electrode."

For some applications, one or both of first connection 3101 and second connection 3102 are provided at least in part by anchor driver 3236. For example, first connection 3101 may be partly provided by a wire 3111 that is electrically connected to tissue-engaging element 3002 (e.g., via an electrically conductive inner core 3004c of the anchor head). Similarly, second connection 3102 may be partly provided by the shaft of anchor driver 3236 (e.g., via an electrical connection between the shaft and outer layer 3004a).

For some applications, wire 3111 may additionally serve as an actuator that controls locking/unlocking of driver 3236 from anchor head 3004. For example, wire 3111 may serve a similar (e.g., identical) function to rod 130, described hereinabove. Similarly, rod 130 may serve as wire 3111, *mutatis mutandis.*

Thus, as is shown in Fig. 9A, during the first phase of the anchoring of an anchor 3006 within the heart, tissue-engaging element 3002 is placed against the surface of tissue 11, such that data-processing system 3521 receives an electrical signal via first connection 3101. Responsively to this signal, the data-processing system provides an indication of the location of the anchor within the heart (e.g., a location along the atrioventricular axis of the heart as described hereinabove). Should the indication be representative of contact with tissue of annulus 10, the second phase of the anchoring process can be initiated, with the anchor being driven into the tissue (Fig. 9B). During this second phase, data-processing system 3521 provides an indication of whether the anchoring process is complete (i.e., of whether anchor-head 3004 is in contact with tissue 11).

For some applications, electrical isolation between the electrodes of anchor head 3004 is provided in order to electrically isolate the signals received such that, during the second phase of anchoring, any signal received via tissue-engaging element 3002 does not preclude determination of contact between the anchor-head and the tissue surface. For some applications, this isolation is provided via an insulating layer 3004b, which may be as described for insulating layer 2004b. Similarly, wire 3111 may be electrically isolated from the shaft of anchor driver 3236 and/or from electrode 3004a (e.g., may extend through an insulated aperture in anchor-head 3004 (e.g. a variant of the insulated aperture described with reference to Fig. 7A-B).

It is to be understood that the first phase and the second phase could be performed sequentially or concurrently by a single data-processing system 3521. Alternatively, two data-processing systems (e.g., data-processing systems 521 and 2521) could be used during the anchoring process, such that first connection 3101 is electrically connected to data-processing system 521 (for use during the first phase of anchoring), and second connection 3102 is connected to data-processing system 2521 (for use during the second phase of anchoring).

A technique for assessing whether an anchor is being driven into tissue of the annulus is now described, in accordance with some applications. For some applications, subsequently to determining that an anchor is contacting tissue of the annulus (e.g. using the methods described hereinabove), it is advantageous to continuously assess, during driving of the anchor into the tissue, whether the anchor is being anchored correctly (e.g. into the correct tissue and/or at a favorable orientation and/or that the anchor is penetrating tissue, and is not suspended in the bloodstream, and/or that the anchor has been driven into the tissue to the required depth). For example, the data-processing system may continue to receive the first signal, the second signal, and/or the refined signal (described hereinabove), and may perform a continuous determination in response to one or more of these signals.

Different tissues of the heart may respond differently to the anchoring process, such that a data-processing system (e.g., as described hereinabove) can provide an indication of the location and/or positioning of the anchor within the tissue, responsively to the electrical signal detected by the anchor as it is driven into the tissue.

The electrical signal detected by the anchor may change as the tissue-engaging element passes into solid tissue, and the resulting electrical signal may therefore be indicative of the location of the anchor within the heart. For example, electrocardiography of the subject during the anchoring process has resulted in the observation that when a tissue anchor is driven into heart tissue of the subject, a temporary electrocardiographic change, e.g., an abnormality such as an ectopic beat, a premature impulse (e.g., a premature ventricular contraction (PVC), and/or a premature atrial contraction (PAC)), typically occurs. The electrocardiographic change typically occurs when while the anchor is placed in contact with the tissue or driven into the tissue, or shortly thereafter (e.g., within 5 seconds and/or 5 heartbeats of the driving of the anchor). The PVC may be initiated by, and indicative of, the distal end of the tissue-engaging element of the tissue anchor penetrating heart muscle tissue (e.g., of the ventricle) after having passed all the way through the annulus. Furthermore, an absence of the abnormality (e.g., the PVC) at the time of anchoring a tissue anchor may be indicative of the tissue-engaging element not having penetrated the heart muscle tissue. For some applications it is advantageous for the distal end of the tissue-engaging element to pass all the way through the annulus.

The technique thus includes using the anchor as an electrode to detect the electrophysiological signals produced by the heart as the anchor is driven into the tissue, and, responsively to the electrical signal, providing an indication of the location of the anchor within the heart. It is to be understood that this indication of the location of the anchor may be (i) an indication regarding the orientation of the anchor within the heart (e.g. the angle-of-attack of the anchor with respect to the tissue into which the anchor is being driven), and/or (ii) an indication regarding the location of the anchor along an atrioventricular axis of the heart, and/or (iii) an indication regarding a plurality of locations through which the anchor is passing as it is being driven into the tissue (e.g. the depth of the anchor within the annulus).

Fig. 10 shows experimental data demonstrating signals received during a period in which the anchor penetrates the tissue of the annulus (time-period t2) are distinguishable from those received during a period in which the anchor merely contacts the surface of the tissue of the annulus (time-period t1). In the experiment, the anchor was held merely in contact with the surface of the tissue during time-period t1, and was subsequently driven into the tissue during time-period t2. Thus, the signals shown in time period t1 of Fig. 10 may generally correspond to the signals shown in Fig. 4D, *mutatis mutandis.* That is, for some applications, Fig. 10 may be considered a temporal extension of Fig. 4D.

It Due to the different electrical responses of the different tissues of the heart to the anchoring process, should the anchor be inadvertently driven into the heart at a suboptimal location and/or angle and/or depth (e.g., other than into the annulus from the atrium), the electrical signal may differ to that shown in Fig. 10. For example, anchoring the anchor into tissue of a leaflet of the valve may not result in such a marked change during the anchoring process. Furthermore, other heart muscle may respond further differently during the anchoring process (e.g. ventricular tissue may respond with a different characterizing electrical signal), such that the data-processing system can provide an indication of the location of the anchor based on the response of the tissue to the anchoring process.

Typically, the data-processing system runs a program in which at least one of the first, second, and refined signals (as described hereinabove) serves as an input, and which responsively determines the location of anchor as it is being driven into the heart.

For some applications, artificial intelligence and/or machine learning is employed in the building of the program. For example, building of the program may be facilitated by the artificial intelligence analyzing data (e.g., a labeled data set) that may include (e.g. refined signals, first signals, second signal, and/or other ECG data obtained from previously performed procedures). For some applications, in order to train (e.g., further train) the program, the location outputted intra-procedurally by the program is compared to the actual location of the anchor (e.g. determined by other means).

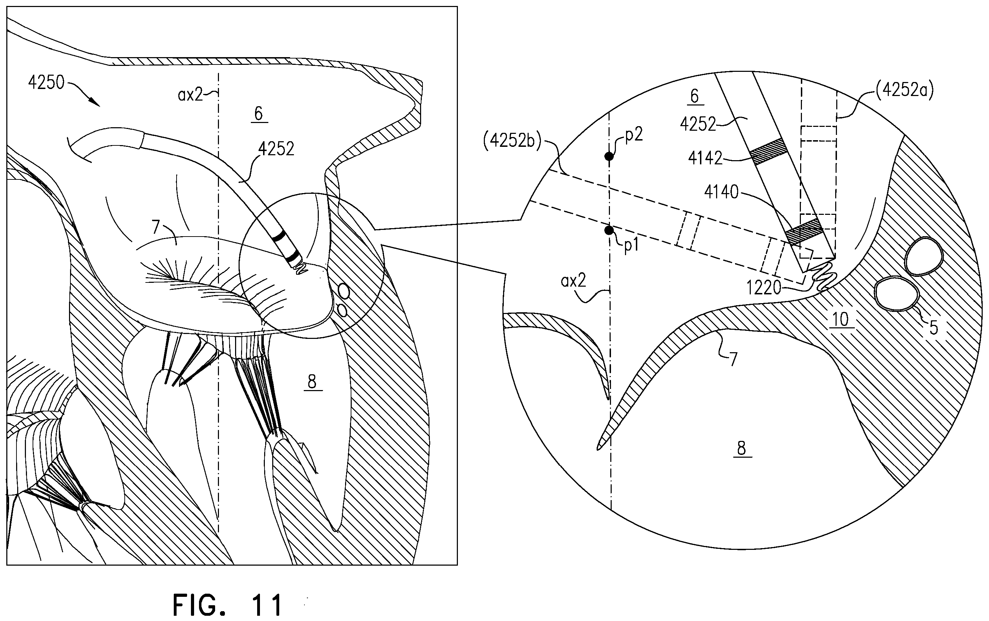

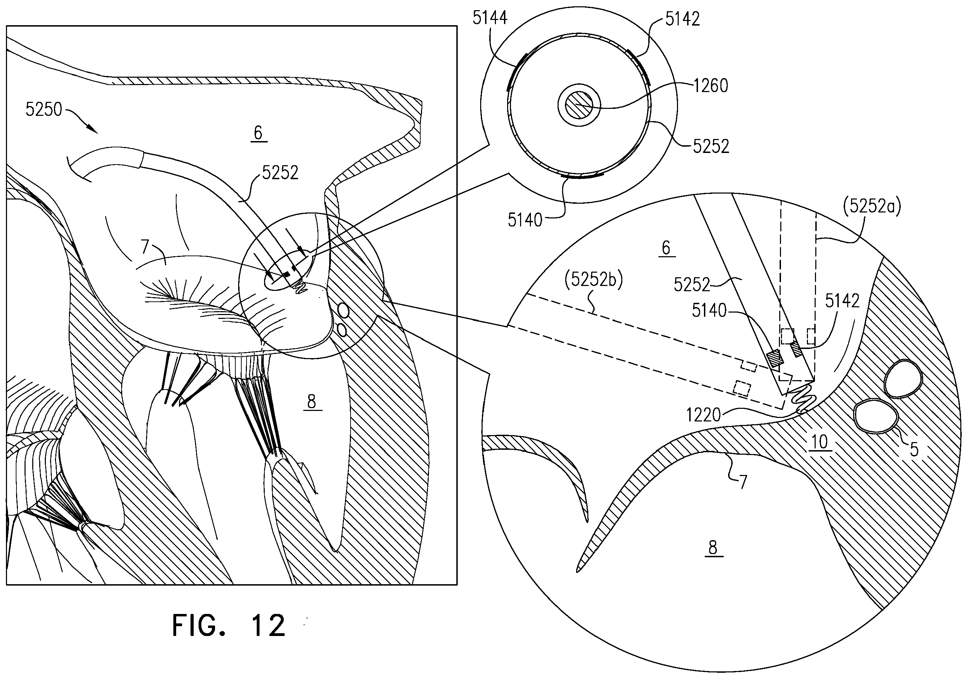

Reference is now made to Figs. 11 and 12, which are schematic illustrations of distal parts of delivery tools 4250 and 5250 that are configured to facilitate determination of their orientation within the heart, in accordance with some applications. In addition, or alternatively, to determining a position of a distal part of a delivery tool and/or an anchor being delivered by the delivery tool (e.g. as described hereinabove), it may be advantageous to determine an orientation (e.g. an angular disposition) of the distal part of the delivery tool within (e.g. with respect to) the heart and/or a tissue thereof. For example, this orientation may control and/or define the angle-of-attack at which the anchor is driven into the tissue. Such an advantage is illustrated in Figs. 11 and 12, in which the distal part of the delivery tool (e.g. of a catheter 4252 or 5252, respectively) is about to drive anchor 1220 into annulus 10. Although, in each case, the tip of anchor 1220 is in contact with annulus 10, optimal anchoring may nonetheless be dependent on the angle of attack at which the anchor is driven into the tissue. This is illustrated by the following examples, which may apply in certain real-world cases, but should be understood to not be limiting:

With reference to Fig. 11, an angle of attack that is excessively shallow with respect to the atrioventricular axis ax2 of the heart may result in anchor 1220 being driven toward a region of tissue (e.g. at the leaflet root) that is thinner than desired, possibly penetrating to the other side of the tissue, and/or resulting in relatively weak anchoring. Such an angle of attack is illustrated by a phantom image of catheter 4252, designated 4252a and labeled in parentheses, which is substantially parallel with atrioventricular axis ax2. Conversely, an angle of attack that is excessively steep with respect to atrioventricular axis ax2 may result in anchor 1220 being driven toward a coronary blood vessel 5, which the anchor may damage, and/or resulting in relatively weak anchoring. Such an angle of attack is illustrated by a phantom image of catheter 4252, designated 4252b and labeled in parentheses, which is nearly orthogonal to atrioventricular axis ax2.

With reference to Fig. 12, an angle of attack that is excessively shallow, in a first direction, with respect to the surface of the tissue may result in anchor 1220 being driven toward into a region of tissue (e.g. at the leaflet root) that is thinner than desired, possibly penetrating to the other side of the tissue, and/or resulting in relatively weak anchoring. Such an angle of attack is illustrated by a phantom image of catheter 5252, designated 5252a and labeled in parentheses, which comes in at an oblique angle from the first direction (e.g. close to the atrial wall behind the leaflet). Conversely, an angle of attack that is excessively shallow, in a second direction, with respect to the surface of the tissue may result in anchor 1220 being driven toward coronary blood vessel 5, which the anchor may damage, and/or resulting in relatively weak anchoring. Such an angle of attack is illustrated by a phantom image of catheter 5252, designated 5252b and labeled in parentheses, which comes in at an oblique angle from the second direction (e.g. close to the plane of the valve).

For each of delivery tools 4250 and 5250, the distal part of the delivery tool comprises multiple electrodes. Each of these delivery tools is electrically connectable to a data-processing system (e.g. a processor) - e.g. may belong to a system that includes the data-processing system, or may simply be compatible with the data-processing system. The data-processing system receives signals from each of the multiple electrodes, responsively to the signals determines an orientation of the distal part of the delivery tool, and provides an output (e.g. a visual and/or audible output) indicative of the orientation. For example, the data-processing system may comprise means, such as hardware and/or software, for performing this method. For some applications, the output is provided via a display, such as described hereinabove. For some applications, the data-processing system is a component of a sensing (and/or procedure-aiding) device, such as described hereinabove. For some applications, the data-processing system is the same data-processing system as one described hereinabove - e.g. a single data-processing system (e.g. a single sensing and/or procedure-aiding device) executes more than one of the techniques described herein.

Although the electrodes of delivery tools 4250 and 5250 are shown and described as being disposed on catheters 4252 and 5252 of the delivery tools (e.g. the catheters out of which anchor 1220 is to be driven), it is to be understood that the scope of the present disclosure includes the electrodes being disposed on other components of the delivery tools, such as, but not limited to, the anchor driver of the delivery tools (i.e. the anchor driver that is engaged with anchor 1220 and that drives the anchor into the tissue - e.g. by applying torque to the anchor). Furthermore, it is to be understood that the scope of the present disclosure includes applying, to delivery tools and systems described elsewhere herein, features described for delivery tools 4250 and 5250 and the systems to which they belong.

At the distal part of delivery tool 4250, multiple electrodes are disposed on catheter 4252. In the example shown, the multiple electrodes are a first electrode 4140 and a second electrode 4142 - but it is to be understood that a greater number of electrodes may be used. Electrodes 4140 and 4142 may be distributed axially along the distal part of the delivery tool (e.g. of the catheter). Each of these electrodes may be a ring electrode that, at its respective axial position, circumscribes the distal part of delivery tool. Described hereinabove, in the context of other systems, are techniques for determining the position of an electrode (e.g. an anchor serving as an electrode) within the heart - e.g. along an atrioventricular axis of the heart. By applying such techniques, *mutatis mutandis,* to determine the position of each of the multiple electrodes, the orientation of the distal part of the delivery tool (e.g. of the catheter) may be determined - e.g. as a straight line that passes through each of the positions.

For some applications, the orientation determined by the data-processing system is an orientation with respect to atrioventricular axis ax2. At least one of the positions determined may be a position along atrioventricular axis ax2 of the heart. In the example shown, a position p1 of first electrode 4140 along atrioventricular axis ax2 is determined, as is a position p2 of the second electrode along the atrioventricular axis. Responsively to positions p1 and p2 (e.g. responsively to a difference (e.g. a distance) therebetween), the data-processing system may determine the orientation of the distal part of delivery tool 4250 (e.g. of catheter 4252) with respect to atrioventricular axis ax2. For example, the determination may be made responsively to (a) the determined difference between the positions of the electrodes within the heart, and (b) a known/predetermined distance between (i) a first electrode-site at which electrode 4140 is disposed on the distal part of the delivery tool, and (ii) a second electrode-site at which electrode 4142 is disposed on the distal part of the delivery tool. A greater determined p1-p2 distance may indicate (e.g. may be determined to represent) a shallower orientation with respect to atrioventricular axis ax2.

At the distal part of delivery tool 5250, multiple electrodes are disposed on catheter 5252. In the example shown, the multiple electrodes are a first electrode 5140, a second electrode 5142, and a third electrode 5144 - but it is to be understood that two electrodes, or four or more electrodes, may be used. Electrodes 5140, 5142, and 5414 may be distributed circumferentially around the distal part of the delivery tool (e.g. of the catheter). Each of these electrodes may be disposed at the same axial position along the distal part of the catheter - e.g. the electrodes may be arranged in a circumferential row.

For some applications, the orientation determined by the data-processing system is an orientation with respect to the surface of the tissue into which anchor 1220 is to be driven. From each of electrodes 5140, 5142, and 5414, a respective signal may be received by the data-processing system. Responsively to the signals, the data-processing system may determine the orientation of the distal part of delivery tool 5250 (e.g. of catheter 5252) with respect to the surface of the tissue. At least one of the signals may be indicative a proximity of the corresponding electrode to a surface of the tissue - e.g. a proximity of the nearest tissue surface, alternatively defined as the shortest distance to a tissue surface. An electrical signal may be applied to the tissue (e.g. between anchor 1220 and each of electrodes 5140, 5142, and 5414), and the data-processing system may determine the orientation of the difference between the signals detected via each of electrodes 5140, 5142, and 5414. For example, the data-processing system may determine a proximity of each of electrodes 5140, 5142, and 5414 to the surface of the tissue, and may determine the orientation responsively to differences between these proximities. A greater difference between the signals may indicate (e.g. may be determined to represent) a shallower orientation with respect to the surface of the tissue, whereas little or no difference may indicate that the distal portion of delivery tool 5250 is transverse (e.g. head on) to the surface of the tissue.

The signal may be (or may be detected as) a bioimpedance signal - e.g. may be indicative of bioimpedance of the tissue(s) through which the electrical signal is driven. This may be applied, for example, based on differences in bioimpedance between blood, annulus tissue, leaflet tissue, and/or myocardium.

Reference is again made to Figs. 11 and 12. The scope of the present disclosure includes combining at least some of the features described with reference to Fig. 11 with at least some of the features described with reference to Fig. 12. For example, a delivery system may comprise, distributed axially along its distal part (thereby providing the axial distribution of Fig. 11), multiple circumferential rows of electrodes (thereby providing the circumferential distribution of Fig. 12). For some applications, the axial and circumferential distributions may both be provided by electrodes distributed helically along and around the distal part of the delivery system, or in another arrangement that includes multiple axial positions and multiple circumferential positions.

Reference is again made to Figs. 1-12. As noted hereinabove, for applications in which intracardiac navigation is facilitated by detection of endogenous (e.g. ECG) signals, a data set (e.g. a labelled data set) may be used - e.g. prior to the procedure, e.g. to build the program that will be run by the data-processing system, such as to train an artificial intelligence and/or machine learning system. Thus, the intra-procedure analysis of the detected endogenous signals may be predicated on the entire data set. Such techniques may be refined using one or more initial (e.g. baseline) signals acquired at the beginning of the procedure - e.g. while the distal part of the delivery tool is distanced from the tissue of the heart. For example, such initial signal(s) may be acquired while the distal part of the delivery tool is disposed in the bloodstream but out of contact with solid tissue, and/or while at a relatively remote site, such as in a vena cava or the aorta. Based on these initial signals, a narrower data set (e.g. a subset of the data set), more appropriate to the particular subject being treated, may be used (directly or indirectly) to facilitate the guidance. For example, the data-processing system may narrow the data set based on one or more parameters of the initial signal(s) such as, but not limited to, heart rate, arrhythmia, and/or shape of the ECG wave - e.g. ratios between various magnitudes and/or intervals, such as the ratio between the P wave and the QRS complex. That is, the narrower data set may be from subjects (real-world subjects and/or simulated subjects) who overall are, with respect to one or more parameters, more similar to and/or representative of the subject being treated than are, overall, those of the entire data set.

For some applications, responsively to the initial signal(s) the data-processing system narrows (e.g. refines) the data set being used (e.g. by categorizing the subject and, based on the categorization, selecting an appropriate narrower data set from a range of narrower data sets). This may be performed as the delivery tool is being advanced to the heart, prior to the actual intracardiac procedure. Subsequently, the intra-procedure analysis of the endogenous signals that facilitates intracardiac navigation is based on the narrower (e.g. selected) data set, and therefore may advantageously be more accurate in its determination of location due to narrower data set being more representative of the subject being treated.

Reference is again made to Figs. 1-12. Although it is noted hereinabove that, for some applications, an exogenous electrical signal may be provided to facilitate sensing, the techniques described hereinabove are typically achieved by detecting only physiological electrical signals (e.g., ECG signals) - e.g. are performed without the application of an exogenous electrical signal to the subject.

Reference is again made to Figs. 1-12. One or more of the detected signals described hereinabove (e.g. one or more of the exogenous signals described hereinabove) may be (or may be detected as) a bioimpedance signal - e.g. may be indicative of bioimpedance of the tissue(s) through which the electrical signal is driven. This may be applied, for example, based on differences in bioimpedance between blood, annulus tissue, leaflet tissue, and/or myocardium. Alternatively or additionally, the use of such bioimpedance signals/measurements may facilitate determination of a degree of contact between a component (e.g. an anchor) and a tissue - e.g. a contact surface area between the component and the tissue.

Reference is again made to Figs. 1-12. For some applications, the data-processing system (e.g. any of the data-processing systems disclosed herein) is, or is a component of, a discrete (e.g. purpose-made) device. For some applications, the data-processing system (e.g. any of the data-processing systems disclosed herein) is a general-purpose data-processing system (e.g. a processor of a general-purpose computer) programmed to run the program.

In the present disclosure, the term data-processing system may refer to, be part of, or include an Application Specific Integrated Circuit (ASIC); a digital, analog, or mixed analog/digital discrete circuit; a digital, analog, or mixed analog/digital integrated circuit; a combinational logic circuit; a field programmable gate array (FPGA); a processor (shared, dedicated, group) that executes code; memory (shared, dedicated, or group) that stores code executed by a processor; other suitable hardware components, such as optical, magnetic, or solid state drives, that provide the described functionality; or a combination of some or all of the above, such as in a system-on-chip. The term code, as used above, may include software, firmware, and/or microcode, and may refer to programs, routines, algorithms, functions, classes, and/or objects. The term shared processor encompasses a single processor that executes some or all code from multiple modules. The term group processor encompasses a processor that, in combination with additional circuitry (e.g. processors), executes some or all code from one or more modules. The term shared memory encompasses a single memory that stores some or all code from multiple modules. The term group memory encompasses a memory that, in combination with additional memories, stores some or all code from one or more modules. The term memory may be subset of the term computer-readable medium. The term computer-readable medium does not encompass transitory electrical and electromagnetic signals propagating through a medium, and may therefore be considered tangible and non-transitory. Non-limiting examples of a non-transitory tangible computer readable medium include nonvolatile memory, volatile memory, magnetic storage, and optical storage.

It is to be noted that, although the apparatus and techniques described herein are shown in the figures on the left side of the heart (e.g. at the mitral valve), the apparatus and techniques described herein may similarly be used on the right side of the heart (e.g. at the tricuspid valve), *mutatis mutandis.*

It is to be noted that, although implants 222 and 1210 are shown as annuloplasty implants, the technology described herein may be used to facilitate implantation of other implants, *mutatis mutandis,* such as other implants that are anchored to the annulus of an atrioventricular valve, e.g., such as some leaflet-restraining and/or leaflet-manipulating implants. For example, the technology described herein may be used to facilitate anchoring of one or more of the implants (e.g., implant 100 and variants thereof) described in International Patent Application PCT/US2021/039587 to Chau et al., which published as WO 2022/006087.

Any of the various systems, devices, apparatuses, etc. in this disclosure can be sterilized (e.g., with heat, radiation, ethylene oxide, hydrogen peroxide, etc.) to ensure they are safe for use with patients, and the methods herein can comprise sterilization of the associated system, device, apparatus, etc. (e.g., with heat, radiation, ethylene oxide, hydrogen peroxide, etc.). Furthermore, the scope of the present disclosure includes, for some applications, sterilizing any of the various systems, devices, apparatuses, etc. in this disclosure.

Although the systems and techniques described herein are generally described as being for use with a human subject, any of the techniques, methods, operations, steps, etc. described or suggested herein can be performed on a non-human animal or on a non-living simulation, such as a cadaver, a cadaver heart, an anthropomorphic ghost, and/or a simulator device (which may include computerized and/or physical representations of body parts, tissue, etc.).

Although the operations of some of the disclosed methods are described in a particular, sequential order for convenient presentation, it should be understood that this manner of description encompasses rearrangement, unless a particular ordering is required by specific language set forth below. For example, operations described sequentially can in some cases be rearranged or performed concurrently. Moreover, for the sake of simplicity, the attached figures may not show the various ways in which the disclosed systems, apparatuses, devices, methods, etc. can be used in conjunction with other systems, apparatuses, devices, methods, etc.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. A system (500) for use with a heart of a subject, the system comprising:
an anchor (32);
a delivery tool (510), configured to deliver the anchor (32) to the heart of the subject, and to drive the anchor (32) into tissue of the heart; and
a data-processing system (521), adapted to, subsequently to the delivery tool (510) delivering the anchor (32) to the heart:
receive, via the delivery tool (510), a first electrical signal from the anchor (32), at the tissue, the anchor (32) serving as an electrode;
receive, via the delivery tool (510), a second electrical signal from a distal part of the delivery tool (510), within the heart,
responsively to both the first electrical signal and the second electrical signal, determine a location of the anchor (32) within the heart, and
provide an output indicative of the location.

2. The system according to claim 1, wherein the data-processing system (521) is adapted to receive the first electrical signal, receive the second electrical signal, determine the location, and provide the output, prior to the delivery tool (510) driving the anchor (32) into the tissue.

3. The system according to any one of claims 1-2, wherein:
the data processing system (521) is adapted to, prior to receiving the first signal:
receive, via the delivery tool (510), in particular prior to the delivery tool (510) delivering the anchor (32) to the heart, a first initial electrical signal from the anchor(32) (32), within the subject but distanced from the tissue, the anchor (32) serving as the electrode,
receive, via the delivery tool (510), in particular prior to the delivery tool (510) delivering the anchor (32) to the heart, a second initial electrical signal from the distal part of the delivery tool, within the subject but distanced from the tissue, and
responsively to the first initial electrical signal and the second initial electrical signal, assign the subject to a category, and
the data-processing system (521) is adapted to determine the location responsively to the first electrical signal, the second electrical signal, and the category.

4. The system according to any one of claims 1-3, wherein:
the output is indicative of the location and an orientation of the anchor (32) within the heart,
the data-processing system (521) is further adapted to, responsively to both the first signal and the second signal:
determine the orientation of the anchor (32) within the heart, and
provide the output indicative of the location and the orientation of the anchor (32) within the heart.

5. The system according to any one of claims 1-4, wherein
the data-processing system (521) is adapted to determine the location of the anchor (32) within the heart by determining a difference between the first signal and the second signal.

6. The system according to any one of claims 1-5, wherein the data-processing system (521) is adapted to, subsequently to providing the output indicative of the location of the anchor (32) within the heart, and while the anchor (32) is driven into the tissue:
responsively to at least one signal selected from the group consisting of the first signal and the second signal, determine a response of the tissue to the anchoring, and
responsively to the determined response, provide an output indicative of at least one of (i) a depth of the anchor (32) within the tissue, and (ii) the location of the anchor (32) within the heart of the subject.

7. The system according to any one of claims 1-6, wherein the anchor (2006) has a tissue-engaging element (2002) and a head (2004), and wherein the data-processing system (521) is adapted to, subsequently to providing the output indicative of the location of the anchor (2006) within the heart, and while the anchor (2006) is driven into the tissue:
responsively to both the first signal and the second signal, determine a response of the tissue to the anchoring,
receive, from the head (2004) of the anchor (2006), a head-contact signal indicative of contact between the head (2004) of the anchor (2006) and the tissue, and
responsively to (i) the determined response, and (ii) the head-contact signal, determine an angle of attack of the anchor (2006) with respect to the tissue.

8. The system according to any one of claims 1-7, wherein:
the delivery tool (510) comprises a driver (36), reversibly engageable with the anchor (32), in particular disengageable from the anchor (32) within the heart, and adapted to drive the anchor (32) into the tissue while engaged with the anchor (32), and
the data-processing system (521) is adapted to receive the first signal via the driver (36).

9. The system according to claim 8, wherein:
the driver (36) comprises:
a tip, reversibly engageable with the anchor (32), and
either
a shaft extending from a proximal end of the driver to the tip, configured to transfer torque from the proximal end of the driver (36) to the tip, and being electrically conductive, and
the data-processing system (521) is adapted to receive the first signal via the shaft;
or
a shaft extending from a proximal end of the driver (36) to the tip, configured to transfer torque from the shaft, and
a rod, extending through the shaft to the tip, configured to control engagement of the tip with the anchor (32), and being electrically conductive, and
the data-processing system (521) is adapted to receive the first signal via the rod.

10. The system according to any one of claims 1-9, wherein:
the system further comprises an adjustment tool (87), and an annuloplasty implant (1210) that comprises:
the anchor (1220), configured to anchor the implant (1210) to the tissue,
a tether (1212), and
a contracting mechanism (40; 1214a, 1214b) configured to, upon actuation of the contracting mechanism, contract the implant, and
the distal part of the delivery tool (1250) comprises a distal part of a guide member of the delivery tool (1250), mechanically connected to the contracting mechanism (40; 1214a, 1214b), the guide member extending (86) proximally from the contracting mechanism (40; 1214a, 1214b), and configured to guide the adjustment tool (87) transluminally to the contracting mechanism (40; 1214a, 1214b) subsequently to anchoring of the implant to the tissue, the adjustment tool (87) being configured to actuate the contracting mechanism (40; 1214a, 1214b), and
the data-processing system (521) is adapted to, subsequently to the delivery tool (510) delivering the anchor (32) to the heart, receive the second electrical signal from the distal part of the guide member that is mechanically connected to the contracting mechanism (40; 1214a, 1214b).

11. The system according to any one of claims 1-10, wherein the anchor (32) defines a distal tip, adapted to penetrate tissue of the heart, and wherein the data-processing system (521) is adapted to receive the first signal while the distal tip of the anchor (32) is placed against the tissue.

12. The system according to any one of claims 1-11, further comprising a sensing device (2520) that comprises the data-processing system (2521) and a first connector, the first connector being electrically and mechanically connectable to a proximal part of the delivery tool, thereby configuring the data-processing system (521) to receive the first signal from the anchor (32) via the first connector.

13. The system according to any one of claims 1-12, wherein:
the heart has an atrium, a ventricle, and a valve therebetween, the valve having an annulus,
the data-processing system (521) is adapted to, subsequently to the delivery tool (510) delivering the anchor (32) to the heart, responsively to both the first signal and the second signal, determine whether the location of the anchor (32) is at the annulus,
the output is indicative of whether the location of the anchor (32) is at the annulus, and
the data-processing system (521) is adapted to provide the output indicative of whether the location of the anchor (32) is at the annulus.

14. The system according to any one of claims 1-13, wherein:
the electrode is a first electrode, the anchor serving as the first electrode, and
the distal part of the delivery tool (510) comprises a second electrode, the data-processing system (521) being adapted to receive the second electrical signal from the second electrode.

15. The system according to any one of claims 1-14, further comprising a reference electrode (536), adapted to be placed outside of the heart of the subject, wherein the data-processing system (521) is configured to determine the location, facilitated by the reference electrode (536).

## Patentansprüche

1. System (500) zur Verwendung mit einem Herzen eines Subjekts, das System umfassend:
einen Anker (32);
ein Zuführwerkzeug (510), das ausgebildet ist, den Anker (32) zu dem Herzen des Subjekts zuzuführen, und den Anker (32) in Gewebe des Herzens einzutreiben; und
ein Datenverarbeitungssystem (521), das angepasst ist, im Anschluss an das Zuführen des Ankers (32) zu dem Herzen durch das Zuführwerkzeug (510):
über das Zuführwerkzeug (510) ein erstes elektrisches Signal von dem Anker (32), an dem Gewebe, zu empfangen, wobei der Anker (32) als eine Elektrode dient;
über das Zuführwerkzeug (510) ein zweites elektrisches Signal von einem distalen Teil des Zuführwerkzeugs (510), innerhalb des Herzens, zu empfangen,
in Reaktion auf sowohl das erste elektrische Signal als auch das zweite elektrische Signal, eine Position des Ankers (32) innerhalb des Herzens zu bestimmen, und
eine Ausgabe bereitzustellen, die die Position angibt.

2. System nach Anspruch 1, wobei das Datenverarbeitungssystem (521) angepasst ist, das erste elektrische Signal zu empfangen, das zweite elektrische Signal zu empfangen, die Position zu bestimmen und die Ausgabe bereitzustellen, bevor das Zuführwerkzeug (510) den Anker (32) in das Gewebe eintreibt.

3. System nach einem der Ansprüche 1-2, wobei:
das Datenverarbeitungssystem (521) angepasst ist, vor dem Empfangen des ersten Signals:
über das Zuführwerkzeug (510), insbesondere vor dem Zuführen des Ankers (32) zu dem Herzen durch das Zuführwerkzeug (510), ein erstes initiales elektrisches Signal von dem Anker (32), innerhalb des Subjekts, jedoch beabstandet von dem Gewebe, zu empfangen, wobei der Anker (32) als die Elektrode dient,
über das Zuführwerkzeug (510), insbesondere vor dem Zuführen des Ankers (32) zu dem Herzen durch das Zuführwerkzeug (510), ein zweites initiales elektrisches Signal von dem distalen Teil des Zuführwerkzeugs, innerhalb des Subjekts, jedoch beabstandet von dem Gewebe, zu empfangen, und
in Reaktion auf das erste initiale elektrische Signal und das zweite initiale elektrische Signal, das Subjekt einer Kategorie zuzuordnen, und
das Datenverarbeitungssystem (521) angepasst ist, die Position in Reaktion auf das erste elektrische Signal, das zweite elektrische Signal und die Kategorie zu bestimmen.

4. System nach einem der Ansprüche 1-3, wobei:
die Ausgabe die Position und eine Ausrichtung des Ankers (32) innerhalb des Herzens angibt,
das Datenverarbeitungssystem (521) weiter angepasst ist, in Reaktion auf sowohl das erste Signal als auch das zweite Signal:
die Ausrichtung des Ankers (32) innerhalb des Herzens zu bestimmen, und
die Ausgabe bereitzustellen, die die Position und die Ausrichtung des Ankers (32) innerhalb des Herzens angibt.

5. System nach einem der Ansprüche 1-4, wobei
das Datenverarbeitungssystem (521) angepasst ist, die Position des Ankers (32) innerhalb des Herzens durch Bestimmen einer Differenz zwischen dem ersten Signal und dem zweiten Signal zu bestimmen.

6. System nach einem der Ansprüche 1-5, wobei das Datenverarbeitungssystem (521) angepasst ist, im Anschluss an das Bereitstellen der Ausgabe, die die Position des Ankers (32) innerhalb des Herzens angibt, und während der Anker (32) in das Gewebe eingetrieben wird:
in Reaktion auf mindestens ein Signal, ausgewählt aus der Gruppe bestehend aus dem ersten Signal und dem zweiten Signal, eine Reaktion des Gewebes auf das Ankern zu bestimmen, und
in Reaktion auf die bestimmte Reaktion, eine Ausgabe bereitzustellen, die mindestens eines von (i) einer Tiefe des Ankers (32) innerhalb des Gewebes, und (ii) der Position des Ankers (32) innerhalb des Herzens des Subjekts angibt.

7. System nach einem der Ansprüche 1-6, wobei der Anker (2006) ein Gewebeeingriffselement (2002) und einen Kopf (2004) aufweist, und wobei das Datenverarbeitungssystem (521) angepasst ist, im Anschluss an das Bereitstellen der Ausgabe, die die Position des Ankers (2006) innerhalb des Herzens angibt, und während der Anker (2006) in das Gewebe eingetrieben wird:
in Reaktion auf sowohl das erste Signal als auch das zweite Signal, eine Reaktion des Gewebes auf das Ankern zu bestimmen,
von dem Kopf (2004) des Ankers (2006) ein Kopfkontaktsignal zu empfangen, das einen Kontakt zwischen dem Kopf (2004) des Ankers (2006) und dem Gewebe angibt, und
in Reaktion auf (i) die bestimmte Reaktion, und (ii) das Kopfkontaktsignal, einen Anstellwinkel des Ankers (2006) in Bezug auf das Gewebe zu bestimmen.

8. System nach einem der Ansprüche 1-7, wobei:
das Zuführwerkzeug (510) einen Treiber (36) umfasst, der reversibel mit dem Anker (32) in Eingriff bringbar ist, insbesondere innerhalb des Herzens von dem Anker (32) lösbar ist, und angepasst ist, den Anker (32) in das Gewebe einzutreiben, während er mit dem Anker (32) in Eingriff steht, und
das Datenverarbeitungssystem (521) angepasst ist, das erste Signal über den Treiber (36) zu empfangen.

9. System nach Anspruch 8, wobei:
der Treiber (36) umfasst:
eine Spitze, die reversibel mit dem Anker (32) in Eingriff bringbar ist, und
entweder
einen Schaft, der sich von einem proximalen Ende des Treibers zu der Spitze erstreckt, der ausgebildet ist, ein Drehmoment von dem proximalen Ende des Treibers (36) zu der Spitze zu übertragen, und elektrisch leitfähig ist, und
das Datenverarbeitungssystem (521) angepasst ist, das erste Signal über den Schaft zu empfangen;
oder
einen Schaft, der sich von einem proximalen Ende des Treibers (36) zu der Spitze erstreckt, der ausgebildet ist, ein Drehmoment von dem Schaft zu übertragen, und
einen Stab, der sich durch den Schaft zu der Spitze erstreckt, der ausgebildet ist, einen Eingriff der Spitze mit dem Anker (32) zu steuern, und elektrisch leitfähig ist, und
das Datenverarbeitungssystem (521) angepasst ist, das erste Signal über den Stab zu empfangen.

10. System nach einem der Ansprüche 1-9, wobei:
das System ferner ein Einstellwerkzeug (87) und ein Anuloplastie-Implantat (1210) umfasst, das umfasst:
den Anker (1220), der ausgebildet ist, das Implantat (1210) an dem Gewebe zu verankern,
eine Leine (1212), und
einen Kontraktionsmechanismus (40; 1214a, 1214b), der ausgebildet ist, bei Betätigung des Kontraktionsmechanismus das Implantat zu kontrahieren, und
der distale Teil des Zuführwerkzeugs (1250) einen distalen Teil eines Führungselements des Zuführwerkzeugs (1250) umfasst, der mechanisch mit dem Kontraktionsmechanismus (40; 1214a, 1214b) verbunden ist, wobei sich das Führungselement (86) proximal von dem Kontraktionsmechanismus (40; 1214a, 1214b) erstreckt, und ausgebildet ist, das Einstellwerkzeug (87) im Anschluss an das Ankern des Implantats an dem Gewebe transluminal zu dem Kontraktionsmechanismus (40; 1214a, 1214b) zu führen, wobei das Einstellwerkzeug (87) ausgebildet ist, den Kontraktionsmechanismus (40; 1214a, 1214b) zu betätigen, und
das Datenverarbeitungssystem (521) angepasst ist, im Anschluss an das Zuführen des Ankers (32) zu dem Herzen durch das Zuführwerkzeug (510), das zweite elektrische Signal von dem distalen Teil des Führungselements zu empfangen, das mechanisch mit dem Kontraktionsmechanismus (40; 1214a, 1214b) verbunden ist.

11. System nach einem der Ansprüche 1-10, wobei der Anker (32) eine distale Spitze definiert, die angepasst ist, Gewebe des Herzens zu penetrieren, und wobei das Datenverarbeitungssystem (521) angepasst ist, das erste Signal zu empfangen, während die distale Spitze des Ankers (32) gegen das Gewebe angelegt ist.

12. System nach einem der Ansprüche 1-11, ferner umfassend eine Erfassungsvorrichtung (2520), die das Datenverarbeitungssystem (2521) und einen ersten Verbinder umfasst, wobei der erste Verbinder elektrisch und mechanisch mit einem proximalen Teil des Zuführwerkzeugs verbindbar ist, wodurch das Datenverarbeitungssystem (521) ausgebildet ist, das erste Signal von dem Anker (32) über den ersten Verbinder zu empfangen.

13. System nach einem der Ansprüche 1-12, wobei:
das Herz einen Vorhof, einen Ventrikel und eine dazwischenliegende Klappe aufweist, wobei die Klappe einen Anulus aufweist,
das Datenverarbeitungssystem (521) angepasst ist, im Anschluss an das Zuführen des Ankers (32) zu dem Herzen durch das Zuführwerkzeug (510), in Reaktion auf sowohl das erste Signal als auch das zweite Signal, zu bestimmen, ob sich die Position des Ankers (32) an dem Anulus befindet,
die Ausgabe angibt, ob sich die Position des Ankers (32) an dem Anulus befindet, und
das Datenverarbeitungssystem (521) angepasst ist, die Ausgabe bereitzustellen, die angibt, ob sich die Position des Ankers (32) an dem Anulus befindet.

14. System nach einem der Ansprüche 1-13, wobei:
die Elektrode eine erste Elektrode ist, wobei der Anker als die erste Elektrode dient, und
der distale Teil des Zuführwerkzeugs (510) eine zweite Elektrode umfasst, wobei das Datenverarbeitungssystem (521) angepasst ist, das zweite elektrische Signal von der zweiten Elektrode zu empfangen.

15. System nach einem der Ansprüche 1-14, weiter umfassend eine Referenzelektrode (536), die angepasst ist, außerhalb des Herzens des Subjekts platziert zu sein, wobei das Datenverarbeitungssystem (521) ausgebildet ist, die Position zu bestimmen, unterstützt durch die Referenzelektrode (536).

## Revendications

1. Système (500) destiné à être utilisé avec le cœur d'un sujet, le système comprenant:
un ancrage (32) ;
un outil de pose (510), conçu pour poser l'ancrage (32) au niveau du cœur du sujet et pour entraîner l'ancrage (32) dans un tissu du cœur ; et
un système de traitement de données (521), adapté à, après la pose de l'ancrage (32) au niveau du cœur par l'outil de pose (510) :
la réception, par l'intermédiaire de l'outil de pose (510), d'un premier signal électrique provenant de l'ancrage (32), au niveau du tissu, l'ancrage (32) servant d'électrode ;
la réception, par l'intermédiaire de l'outil de pose (510), d'un second signal électrique provenant d'une partie distale de l'outil de pose (510), à l'intérieur du cœur,
en réponse à la fois au premier signal électrique et au second signal électrique, la détermination d'un emplacement de l'ancrage (32) à l'intérieur du cœur et
la fourniture d'une sortie indiquant l'emplacement.

2. Système selon la revendication 1, le système de traitement de données (521) étant adapté à la réception du premier signal électrique, la réception du second signal électrique, la détermination de l'emplacement et la fourniture de la sortie, avant l'entraînement de l'ancrage (32) dans le tissu par l'outil de pose (510).

3. Système selon l'une quelconque des revendications 1 à 2 :
le système de traitement de données (521) étant adapté à, avant la réception du premier signal :
la réception, par l'intermédiaire de l'outil de pose (510), en particulier avant la pose de l'ancrage (32) au niveau du cœur par l'outil de pose (510), d'un premier signal électrique initial provenant de l'ancrage (32), à l'intérieur du sujet mais à distance du tissu, l'ancrage (32) servant d'électrode,
la réception, par l'intermédiaire de l'outil de pose (510), en particulier avant la pose de l'ancrage (32) au niveau du cœur par l'outil de pose (510), d'un second signal électrique initial provenant de la partie distale de l'outil de pose, à l'intérieur du sujet mais à distance du tissu et
en réponse au premier signal électrique initial et au second signal électrique initial, l'attribution du sujet à une catégorie et
le système de traitement de données (521) étant adapté à la détermination de l'emplacement en réponse au premier signal électrique, au second signal électrique et à la catégorie.

4. Système selon l'une quelconque des revendications 1 à 3 :
la sortie indiquant l'emplacement et une orientation de l'ancrage (32) à l'intérieur du cœur,
le système de traitement de données (521) étant en outre adapté à, en réponse à la fois au premier signal et au second signal :
la détermination de l'orientation de l'ancrage (32) à l'intérieur du cœur et
la fourniture de la sortie indiquant l'emplacement et l'orientation de l'ancrage (32) à l'intérieur du cœur.

5. Système selon l'une quelconque des revendications 1 à 4,
le système de traitement de données (521) étant adapté à la détermination de l'emplacement de l'ancrage (32) à l'intérieur du cœur par détermination d'une différence entre le premier signal et le second signal.

6. Système selon l'une quelconque des revendications 1 à 5, le système de traitement de données (521) étant adapté à, après la fourniture de la sortie indiquant l'emplacement de l'ancrage (32) à l'intérieur du cœur et pendant que l'ancrage (32) est entraîné dans le tissu :
en réponse à au moins un signal choisi dans le groupe constitué par le premier signal et le second signal, la détermination d'une réponse du tissu à l'ancrage et
en réponse à la réponse déterminée, la fourniture d'une sortie indiquant au moins un élément parmi (i) une profondeur de l'ancrage (32) à l'intérieur du tissu et (ii) l'emplacement de l'ancrage (32) à l'intérieur du cœur du sujet.

7. Système selon l'une quelconque des revendications 1 à 6, l'ancrage (2006) présentant un élément de mise en prise (2002) avec le tissu et une tête (2004) et le système de traitement de données (521) étant adapté à, après la fourniture de la sortie indiquant l'emplacement de l'ancrage (2006) à l'intérieur du cœur et pendant que l'ancrage (2006) est entraîné dans le tissu :
en réponse à la fois au premier signal et au second signal, la détermination d'une réponse du tissu à l'ancrage,
la réception, en provenance de la tête (2004) de l'ancrage (2006), d'un signal de contact de tête indiquant un contact entre la tête (2004) de l'ancrage (2006) et le tissu et
en réponse (i) à la réponse déterminée et (ii) au signal de contact de tête, la détermination d'un angle d'attaque de l'ancrage (2006) par rapport au tissu.

8. Système selon l'une quelconque des revendications 1 à 7 :
l'outil de pose (510) comprenant un dispositif d'entraînement (36), pouvant venir en prise de manière réversible avec l'ancrage (32), en particulier pouvant être séparé de l'ancrage (32) à l'intérieur du cœur, et adapté à l'entraînement de l'ancrage (32) dans le tissu tout en étant en prise avec l'ancrage (32) et
le système de traitement de données (521) étant adapté à la réception du premier signal par l'intermédiaire du dispositif d'entraînement (36).

9. Système selon la revendication 8 :
le dispositif d'entraînement (36) comprenant :
une pointe, pouvant venir en prise de manière réversible avec l'ancrage (32) et
soit
une tige s'étendant à partir d'une extrémité proximale du dispositif d'entraînement à la pointe, conçue pour transférer un couple à partir de l'extrémité proximale du dispositif d'entraînement (36) à la pointe et étant électriquement conductrice et
le système de traitement de données (521) étant adapté à la réception du premier signal par l'intermédiaire de la tige ;
soit
une tige s'étendant à partir d'une extrémité proximale du dispositif d'entraînement (36) à la pointe, conçue pour transférer un couple à partir de la tige et
une barre, s'étendant à travers la tige à la pointe, conçue pour commander la mise en prise de la pointe avec l'ancrage (32) et étant électriquement conductrice et
le système de traitement de données (521) étant adapté à la réception du premier signal par l'intermédiaire de la barre.

10. Système selon l'une quelconque des revendications 1 à 9 :
le système comprenant en outre un outil d'ajustement (87) et un implant d'annulo-plastie (1210) qui comprend :
l'ancrage (1220), conçu pour ancrer l'implant (1210) au tissu,
une attache (1212) et
un mécanisme de contraction (40 ; 1214a, 1214b) conçu pour, lors de l'actionnement du mécanisme de contraction, contracter l'implant et
la partie distale de l'outil de pose (1250) comprenant une partie distale d'un élément de guidage de l'outil de pose (1250), reliée mécaniquement au mécanisme de contraction (40 ; 1214a, 1214b), l'élément de guidage s'étendant (86) de manière proximale à partir du mécanisme de contraction (40 ; 1214a, 1214b) et étant conçu pour guider l'outil d'ajustement (87) de manière transluminale vers le mécanisme de contraction (40 ; 1214a, 1214b) après l'ancrage de l'implant au tissu, l'outil d'ajustement (87) étant conçu pour actionner le mécanisme de contraction (40 ; 1214a, 1214b) et
le système de traitement de données (521) étant adapté à, après la pose de l'ancrage (32) au niveau du cœur par l'outil de pose (510), la réception du second signal électrique provenant de la partie distale de l'élément de guidage qui est reliée mécaniquement au mécanisme de contraction (40 ; 1214a, 1214b).

11. Système selon l'une quelconque des revendications 1 à 10, l'ancrage (32) définissant une pointe distale, adapté à la pénétration dans un tissu du cœur et le système de traitement de données (521) étant adapté à recevoir le premier signal tandis que la pointe distale de l'ancrage (32) est placée contre le tissu.

12. Système selon l'une quelconque des revendications 1 à 11, comprenant en outre un dispositif de détection (2520) qui comprend le système de traitement de données (2521) et un premier connecteur, le premier connecteur pouvant être connecté électriquement et mécaniquement à une partie proximale de l'outil de pose, ce qui permet de configurer le système de traitement de données (521) pour recevoir le premier signal provenant de l'ancrage (32) par l'intermédiaire du premier connecteur.

13. Système selon l'une quelconque des revendications 1 à 12 :
le cœur présentant un atrium, un ventricule et une valvule entre eux, la valvule présentant un anneau,
le système de traitement de données (521) étant adapté à, après la pose de l'ancrage (32) au niveau du cœur par l'outil de pose (510), en réponse à la fois au premier signal et au second signal, la détermination du fait que l'emplacement de l'ancrage (32) se trouve au niveau de l'anneau ou non,
la sortie indiquant si l'emplacement de l'ancrage (32) se trouve au niveau de l'anneau ou non et
le système de traitement de données (521) étant adapté à la fourniture de la sortie indiquant si l'emplacement de l'ancrage (32) se trouve au niveau de l'anneau ou non.

14. Système selon l'une quelconque des revendications 1 à 13 :
l'électrode étant une première électrode, l'ancrage servant de première électrode et
la partie distale de l'outil de pose (510) comprenant une seconde électrode, le système de traitement de données (521) étant adapté à la réception du second signal électrique provenant de la seconde électrode.

15. Système selon l'une quelconque des revendications 1 à 14, comprenant en outre une électrode de référence (536), adaptée au placement à l'extérieur du cœur du sujet, le système de traitement de données (521) étant configuré pour déterminer l'emplacement, ce qui est facilité par l'électrode de référence (536).
